# FASCICULE DE BREVET EUROPEEN

(11) **EP 0 230 179 B1**
(45) Date de publication et mention de la délivrance du brevet: **30.09.1992**
(21) Numéro de dépôt: 86402764.4
(22) Date de dépôt: 11.12.1986
(51) Int. Cl.: C07D 413/04, C07D 209/08, A61K 31/535

(54) **Nouveaux dérivés du 4-morpholinyl 1H-indole, leurs sels, procédé et intermédiaires de préparation, application à titre de médicaments, compositions les renfermant**
4-Morpholinyl 1H-indol-Derivate, ihre Salze, Verfahren und Zwischenprodukte zur Herstellung, Verwendung als Arzneimittel, sie enthaltende Zusammensetzungen
4-Morpholinyl 1H-indole derivatives, their salts, process and intermediates of preparation, use as therapeutic agents, compositions containing them

(30) Priorité: 13.12.1985 FR 8518482
(43) Date de publication de la demande: 29.07.1987
(73) Titulaire: ROUSSEL-UCLAF, 75007 Paris (FR)
(72) Inventeur: Gasc, Jean-Claude, F-93140 Bondy (FR); Nedelec, Lucien, F-93340 Le Raincy (FR); Rettien, Claude, F-93100 Montreuil (FR); Weissmann Nanopoulos, Dinah, F-69002 Lyon (FR)
(74) Mandataire: Vieillefosse, Jean-Claude

(56) Documents cités:
- FR-A- 2 501 208
- FR-A- 2 512 817
- JOURNAL OF HETEROCYCLIC CHEMISTRY, vol. 20, septembre/octobre 1983; R.D.CLARK, pp. 1393-1395

## Description

La présente invention concerne de nouveaux dérivés du 4-morpholinyl 1 H-indole, ainsi que leurs sels, le procédé de préparation, l'application à titre de médicaments de ces nouveaux dérivés, les compositions les renfermant et des intermédiaires.

Dans le brevet français BF 2.501.208 sont décrits des dérivés du morpholinyl indole et dans le brevet français BF 2.512.817 sont décrits des dérivés de l'aminométhyl 1 H-indole-4-méthanol.

L'invention a pour objet de nouveaux dérivés du 4-morpholinyl 1 H-indole, ainsi que leurs sels d'addition avec les acides minéraux ou organiques, caractérisés en ce qu'ils répondent à la formule générale (1) : 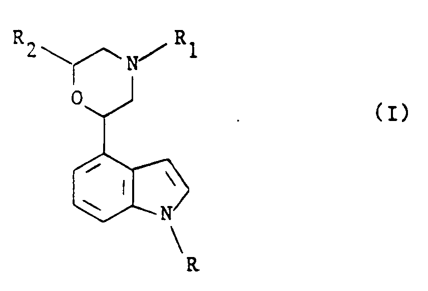 dans laquelle R représente un atome d'hydrogène ou un radical alkyle renfermant de 1 à 4 atomes de carbone, R₁ représente un atome d'hydrogène, un radical alkyle renfermant de 1 à 4 atomes de carbone, R₂ représente un radical hydroxyméthyle, alkylthiométhyle dont l'alkyle comprend de 1 à 3 atomes de carbone, cyanométhyle ou carboxy éventuellement estérifié par un alcool aliphatique renfermant de 1 à 5 atomes de carbone, ou amidifié par une amine de formule 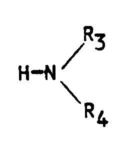 dans laquelle R₃ représente un atome d'hydrogène ou un radical alkyle renfermant de 1 à 4 atomes de carbone, et R₄ représente un radical alkyle renfermant de 1 à 4 atomes de carbone.

Dans la formule générale (I) et dans ce qui suit, le terme radical alkyle renfermant de 1 à 4 atomes de carbone désigne de préférence un radical méthyle, éthyle, propyle ou isopropyle ; le terme alcool aliphatique renfermant de 1 à 5 atomes de carbone désigne de préférence le méthanol, l'éthanol, le propanol ou l'isopropanol ; le terme alkylthiométhyle désigne par exemple le radical n-propyl thiométhyle, éthylthiométhyle ou de préférence méthylthiométhyle.

Les sels d'addition avec les acides minéraux ou organiques peuvent être, par exemple, les sels formés avec les acides chlorhydrique, bromhydrique, nitrique, sulfurique, phosphorique, acétique, formique, propio- nique, benzoïque, maléique, fumarique, succinique, tartrique, citrique, oxalique, glyoxylique, aspartique, alcane sulfoniques tels que les acides méthane-et éthane-sulfoniques, arylsulfoniques, tels que les acides benzène-et paratoluène-sulfoniques et arylcarboxyliques.

Parmi les produits, objet de l'invention, on peut citer les dérivés répondant à la formule 1 ci-dessus, ainsi que leurs sels d'addition avec les acides minéraux ou organiques, caractérisés en ce que, dans ladite formule I, R représente un atome d'hydrogène, notamment ceux pour lesquels R₂ représente un radical hydroxyméthyle, alkyl thiométhyle dont l'alkyle comprend de 1 à 3 atomes de carbone ou cyano-méthyle ou un radical carboxy éventuellement amidifié par une amine de formule 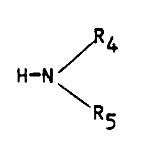 dans laquelle R₄ et R₅ représentent un radical alcoyle linéaire renfermant de 1 à 4 atomes de carbone.

Parmi ceux-ci, on peut citer plus particulièrement les dérivés caractérisés en ce que, dans ladite formule (1), R₂ représente un radical alkyl thiométhyle dont l'alkyle comprend de 1 à 3 atomes de carbone et tout particulièrement :
- le 4-/(2R-cis) 4-méthyl 6-/(méthylthio) méthyl/ 2-morpholinyl/ 1 H-indole ainsi que ses sels d'addition avec les acides minéraux ou organiques.

Les dérivés racémiques de formule l aussi bien que les isomères optiquement actifs entrent dans le cadre de l'invention.

L'invention a également pour objet un procédé de préparation des dérivés, tels que définis par la formule 1 ci-dessus, aussi que de leurs sels, caractérisé en ce que l'on soumet à une déshydrogénation un dérivé de formule (II) : 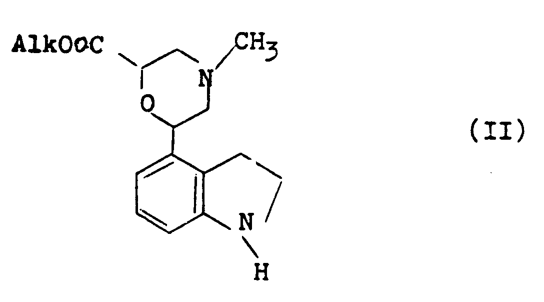 dans laquelle Alk représente un radical alkyle renfermant de 1 à 4 atomes de carbone, pour obtenir un dérivé de formule (I_{A}) : 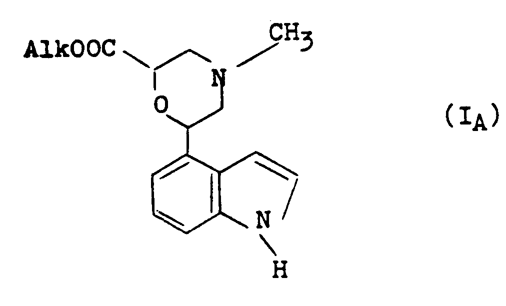 dans laquelle Alk a la signification déjà indiquée, que
- soit l'on isole et si désiré, salifie ;
- soit l'on déméthyle pour obtenir le produit de formule (I_{B}) : 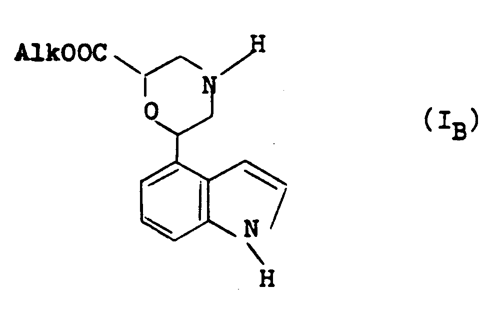 dans laquelle Alk a la signification déjà indiquée, que
- soit l'on isole et si désiré, salifie ;
- soit l'on fait réagir avec un agent d'alkylation, pour obtenir un produit de formule (l_{c}) : 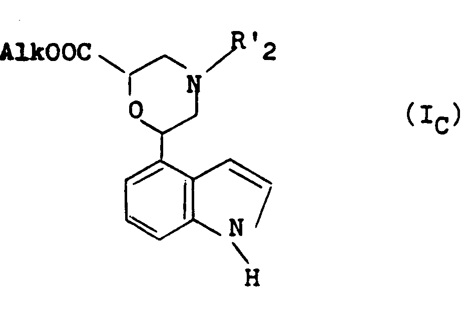 dans laquelle R'₂ a a la signification de R₂ déjà indiquée à l'exception de l'hydrogène et alk a la signification déjà indiquée, que ou bien l'on isole, et, si désiré, salifie, ou bien l'on fait réagir avec un amidure alcalin, puis avec un halogénure d'alkyle de formule (IV): 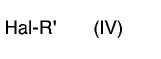 dans laquelle Hal représente un atome de chlore, de brome ou d'iode et R' représente un radical alkyle renfermant de 1 à 4 atomes de carbone, pour obtenir un produit de formule (I_{D}) : 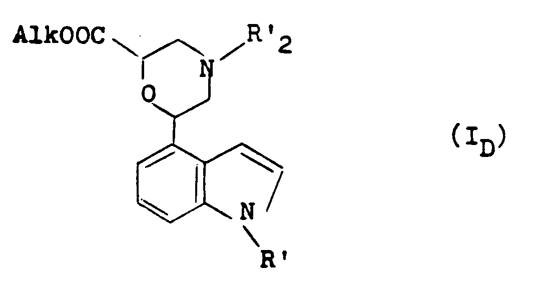 dans laquelle Alk, R' et R'₂ ont la signification déjà indiquée, que l'on isole et, si désiré, salifie ;
- soit l'on saponifie ledit produit de formule (I_{A}) pour obtenir un produit de formule (I_{E}): 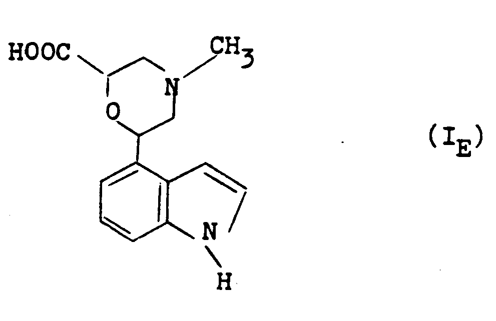 que ou bien l'on isole et, si désiré, salifie, ou bien l'on fait réagir avec une amine de formule (V) : 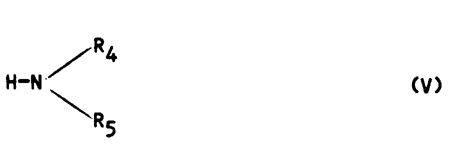 dans laquelle R₄ et R₅ ont la signification déjà indiquée, pour obtenir un produit de formule (I_{F}) : 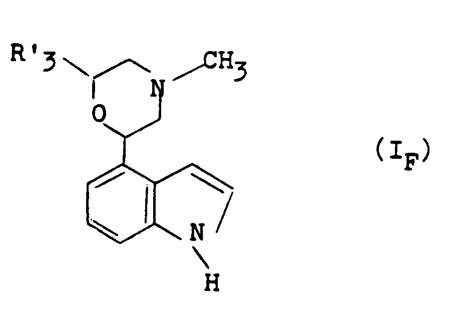 dans laquelle R'₃ représente un radical carboxy amidifié par l'amide de formule (V) définie ci-dessus, que l'on isole et si désiré, salifie ;
   soit réduit ledit produit de formule (I_{A}) pour obtenir un produit de formule (I_{G}) : 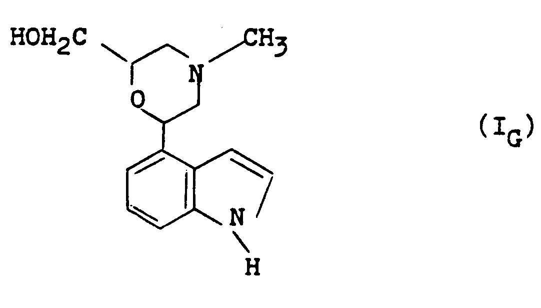 que ou bien l'on isole et, si désiré, salifie, ou bien l'on fait réagir avec le chlorure de méthane ou de paratoluène sulfonyle, pour obtenir un produit de formule (VI) : 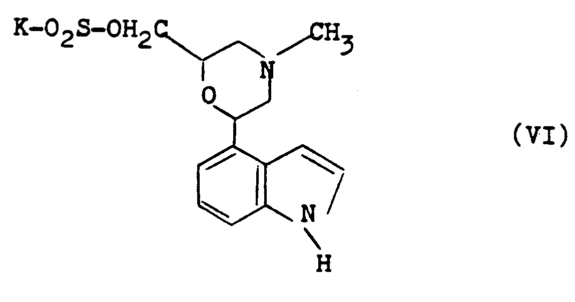 dans laquelle K représente un radical méthyle ou p-tolyle, que l'on fait réagir avec un alkylmercaptan ou avec un cyanure alcalin, pour obtenir un produit de formule (I_{H}) : 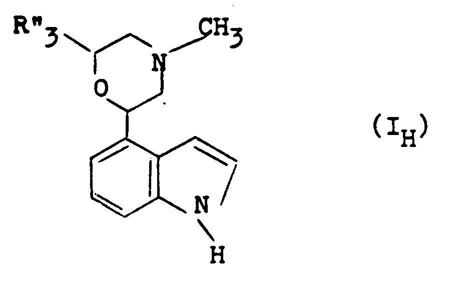 dans laquelle R"₃ représente un radical alkylthiométhyle ou cyanométhyle, que l'on isole et si désiré, salifie, puis effectue ensuite si désiré, sur les produits de formules (I_{E}), (I_{F}), (l_{G}) et (I_{H}), une ou plusieurs des réactions déjà indiquées ci-dessus pour les produits de formule (I_{A}), pour obtenir les produits correspondants entrant dans la formule (1), puis isole, et si désiré, salifie les produits ainsi obtenus.

Dans des conditions préférentielles de mise en oeuvre de l'invention, le procédé de préparation ci-dessus décrit est mis en oeuvre dans les conditions indiquées ci-après :
- La déshydrogénation du dérivé de formule Il est réalisée de préférence par action de bioxyde de manganèse. On peut également utiliser l'oxygène en présence d'un métal tel que le palladium ou le sélénium.
- La déméthylation du produit de formule (I_{A}) est réalisée de préférence par action du bromure de cyanogène, suivie d'une réduction telle qu'obtenue par action du zinc dans l'acide acétique.
- L'alkylation du dérivé de formule (l_{B}) est réalisée de préférence à l'aide d'un halogénure d'alcoyle, notamment un iodure d'alcoyle, en présence d'un agent de condensation tel qu'un carbonate alcalin.
- L'halogénure de formule (IV) peut être un chlorure ou un bromure, mais de préférence un iodure ; on le fait réagir après action, notamment dans l'ammoniaque, d'un amidure alcalin, de préférence d'un amidure de sodium, sur le produit de formule (l_{c}).
- La saponification du produit de formule (I_{A}) est réalisée de préférence par action d'une base forte telle que la soude 2N, ou de préférence un alcoolate alcalin tel que le méthylate de sodium ; on peut également opérer une hydrolyse en milieu acide, par exemple par action d'un acide minéral tel que l'acide chlorhydrique dilué, dans un solvant tel qu'un alcanol renfermant de 1 à 5 atomes de carbone, notamment l'éthanol.
- La réaction du produit de formule (I_{E}) avec l'amine de formule (V) pour conduire au produit de formule (I_{F}) est réalisée de préférence après activation de la fonction acide carboxylique par formation d'un anhydride mixte, par exemple, par action d'anhydride trifluoroacétique ou d'un halogénoformiate d'alcoyle tel que le chloroformiate d'isobutyle.
- La réduction du produit de formule (I_{A}) est réalisée de préférence par action de borohydrure de sodium, au reflux d'un solvant tel que dioxanne ou le mélange dixoanne-méthanol ou dioxanne- éthanol ; on peut également utiliser d'autres réducteurs tel le cyano-borohydrure de sodium.
- La réaction du produit de formule (l_{G}) avec le chlorure de méthane ou de paratoluène sulfonyle est réalisée de préférence dans la pyridine à température ambiante.
- La réaction du produit de formule (VI) avec l'alcoylmercaptan est réalisée de préférence à température ambiante dans un solvant tel que le diméthylacétamide, en présence d'hydrure de sodium ; l'alcoylmercaptan est de préférence le méthylmercaptan. Le cyanure alcalin que l'on fait réagir avec le produit de formule (VI) est de préférence le cyanure de sodium ou de potassium ; on opère avantageusement dans un solvant tel que le diméthylformamide.

Les dérivés de formule (1), à l'exception des dérivés de formule (I) dans laquelle R₂ représente un radical carboxy libre, présentent un caractère basique. On peut avantageusement préparer les sels d'addition des dérivés de formule (1), en faisant réagir, en proportions sensiblement stoechiométriques, un acide minéral ou organique avec ledit dérivé de formule (1). Les sels peuvent être préparés sans isoler les bases correspondantes.

Les dérivés de formule Il peuvent être préparés, par exemple, par réaction d'un produit de formule VII : 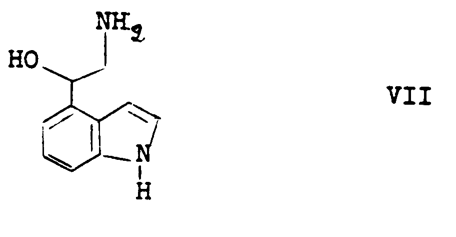 avec un halogénoformiate de méthyle doht l'halogène est le brome, ou, de préférence, le chlore, pour obtenir un produit de formule VIII : 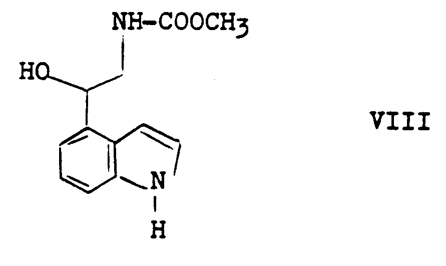 que l'on réduit, de préférence à l'aide du complexe boranetriméthylamine, pour obtenir un produit de formule IX : 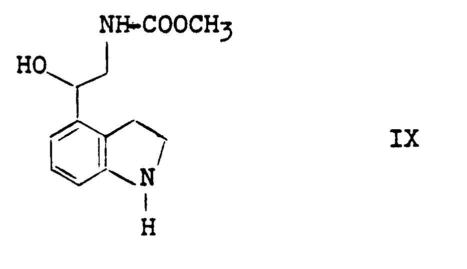 que l'on fait réagir avec un halogénure, de préférence un chlorure de benzoyle, pour obtenir un produit de formule X : 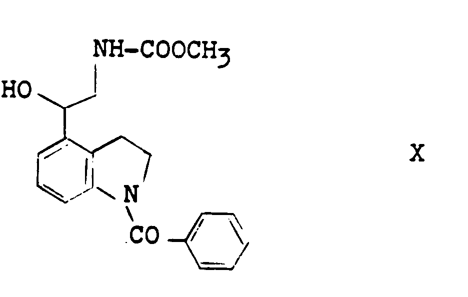 que l'on réduit, de préférence à l'aide d'hydrure d'aluminium-lithium, pour obtenir un produit de formule XI : 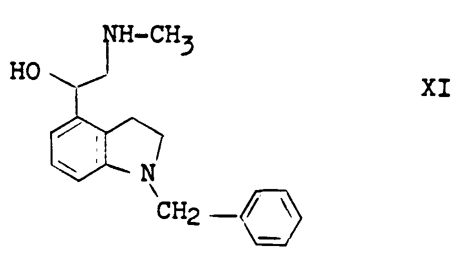 que l'on fait réagir avec un glycidate d'alkyle de formule XII : 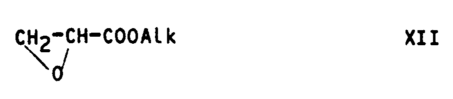 dans laquelle Alk a la signification déjà indiquée, pour obtenir un produit de formule XIII : 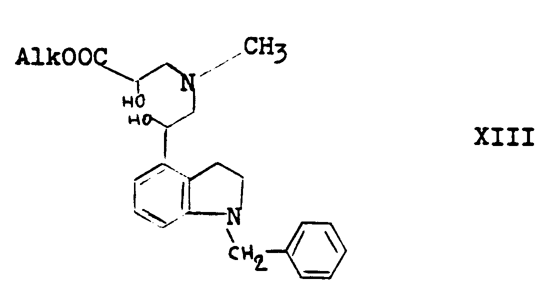 dans laquelle Alk a la signification déjà indiquée, que l'on cyclise, par exemple par action de N-chloro diisopropylamine en présence d'hexaméthyl phosphoramine ou, de préférence, de tris diméthylaminophos- phine, pour obtenir un produit de formule XIV : 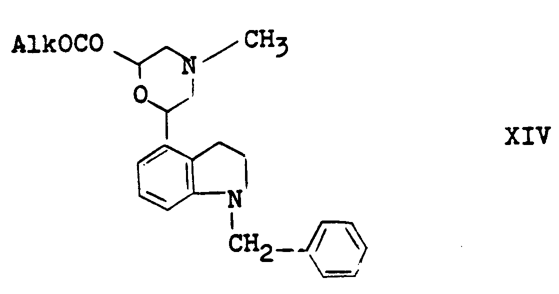 dans laquelle Alk a la signification déjà indiquée, que l'on débenzyle, par exemple, par hydrogénation catalytique, pour obtenir le produit de formule Il recherché.

Les dérivés, objet de la présente invention, possèdent de très intéressantes propriétés pharmacologiques. Ils protègent les neurones contre les agressions ischémiques ou anoxiques et assurent leur activation. Ils améliorent de plus les processus cognitifs.

Ces propriétés sont illustrées plus loin dans la partie expérimentale.

Ces propriétés justifient l'utilisation des dérivés du 4-morpholinyl 1H-indole de formule I, ainsi que de leurs sels, à titre de médicaments.

La présente demande a ainsi également pour objet l'application, à titre de médicaments, des dérivés du 4-morpholinyl 1 H-indole tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du 4-morpholinyl 1 H-indole répondant à la formule I, dans laquelle R représente un atome d'hydrogène ainsi que par leurs sels d'addition avec les acides pharmaceutiquement acceptables.

Parmi ceux-ci, on retient notamment ceux répondant à la formule I, dans laquelle R₂ représente un radical hydroxyméthyle, alkyl thiométhyle ou cyano-méthyle ou un radical carboxy éventuellement amidifié par une amine de formule 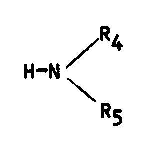 dans laquelle R₄ et R₅ représentent un radical alcoyle linéaire renfermant de 1 à 4 atomes de carbone et plus particulièrement R₂ représente un radical alkylthiométhyle ainsi que leurs sels d'addition avec les acides pharmaceutiquement acceptables.

Parmi ces derniers, on retient tout particulièrement, le dérivé dont le nom suit :
- le 4-/(2R-cis) 4-méthyl 6-/(méthylthio) méthyl/ 2-morpholinyl/ 1 H-indole ainsi que ses sels d'addition avec les acides pharmaceutiquement acceptables.

Les médicaments selon l'invention trouvent leur emploi dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé ou atteint d'artériosclérose et dans le traitement de l'hypertension d'origine rénale. Ils peuvent aussi être utilisés dans le traitement de la dégénérescence et de la sénescence cérébrale ou des manifestations liées à un hypoxie cérébrale.

La dose usuelle, variable selon le dérivé utilisé, le sujet et l'affection en cause peut être par exemple, de 5 mg à 200 mg par jour. Par voie orale, chez l'homme, le dérivé de l'exemple 3 peut être administré à la dose quotidienne de 5 mg à 200 mg par jour. Par voie orale, chez l'homme, le dérivé de l'exemple 3 peut être administré à la dose quotidienne de 5 mg à 50 mg, par exemple pour le traitement de la sénescence cérébrale, soit environ de 0,07 mg à 0,7 mg par kilogramme de poids corporel.

L'invention a enfin pour objet les compositions pharmamceutiques qui renferment au moins un dérivé précité ou l'un de ses sels d'addition avec les acides pharmaceutiquement acceptables, à titre de principe actif.

A titre de médicaments, les dérivés répondant à la formule 1 et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux dérivés utiles notamment pour la préparation des dérivés répondant à la formule (1), à savoir les produits de formule (XIII) : 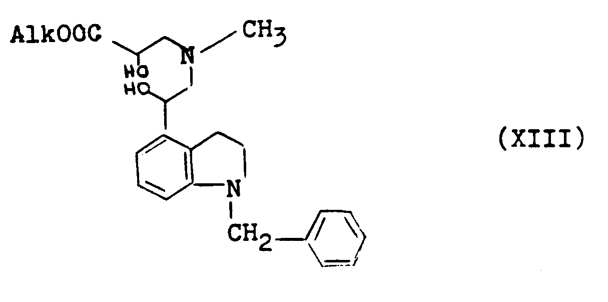 dans laqu elle Alk a la signification déjà indiquée.

Les exemples qui suivent illustrent la présente invention, sans toutefois la limiter.

### Exemple 1 : (2 RS, 6 RS) 6-(1H-indol 4-yl) 4-méthyl 2-morpholine carboxylate d'éthyle.

On dissout 3,45 g de (2RS,6RS) 6-(2,3-dihydro 1 H-indol-4-yl) 4-méthyl 2-morpholine carboxylate d'éthyle dans 345 cm3 de chlorure de méthylène, ajoute 13,8 g de bioxyde de manganèse actif, agite pendant 30 mn, filtre, lave au chlorure de méthylène, amène à sec sous pression réduite, et obtient 3,185 g du produit attendu.

### Exemple 2 : N,N-diéthyl 6-(1H-indol 4-yl) 4-méthyl 2-morpholine carboxamide et son mésylate

### Stade A/ Saponification :

On porte sous agitation pendant 65 h sous atmosphère inerte 4,5 g de (2RS, 6RS) 6-(1 H-indol 4-yl) 4-méthyl 2-morpholine carboxylate d'éthyle dans 45 cm3 de méthanol avec 31,21 cm3 de solution 0,5 M de méthylate de sodium dans le méthanol, amène à sec et obtient 4,33 g du sel de sodium de l'acide, que l'on utilise tel quel pour l'étape suivante.

### Stade B/ Amidification :

On porte sous agitation et atmosphère inerte les 4,33 g de produit ci-dessus dans 75 cm3 de dioxanne et 90 cm3 de diméthylformamide, ajoute à 10αC 5,48 cm3 de tributylamine puis, goutte à goutte 3 cm3 de chloroformiate d'isobutyle, après 1 H 30mn d'agitation ajoute goutte à goutte 7,93 cm3 de diéthylamine, agite 4H 30mn, et amène à sec sous pression réduite. On reprend à l'eau le résidu sec, extrait au chloroforme, lave à l'eau, sèche, évapore à sec, chromatographie le résidu sur silice (Eluant : chlorure de méthylène-isopropanol 9/1), traite au charbon actif, chromatographie sur silice (Eluant : chlorure de méthylène-isopropanol 85-15) et obtient 0,888 g de la base attendue.

### Stade C/ Formation du mésylate :

On dissout le produit obtenu ci-dessus dans l'isopropanol, ajoute 2,82 cm3 d'une solution 1 M/1 d'acide méthane sulfonique dans l'isopropanol, concentre légèrement, ajoute 8 cm3 d'éther isopropylique, et obtient 1,107 mg du produit attendu F◊ ≃ 100-115◊C.

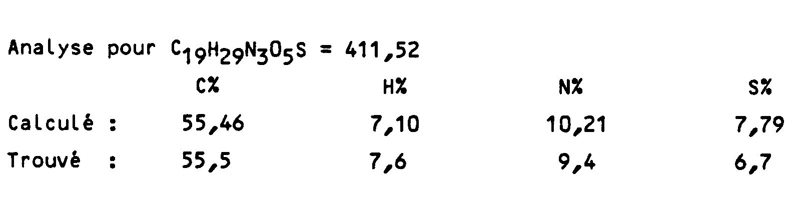

### Exemple 3 : (2 RS, 6 RS) 6-(1H-indol 4-yl) 4-méthyl 2-morpholine méthanol, et son fumarate neutre

On ajoute 3,185 g de borhydure de sodium à une solution de 3,185 g du produit obtenu au stade B/ de l'exemple 2 dans 40 cm3 de méthanol et 20 cm3 de dioxanne, agite 1 heure au reflux, refroidit et dilue avec 400 cm3 d'eau. On extrait au chlorure de méthylène, sèche, amène à sec sous pression réduite, chromatographie sur silice (Eluant : chlorure de méthylène-méthanol 88-12), et obtient 2,105 g de produit attendu.

### Formation du fumarate neutre.

On dissout le produit obtenu ci-dessus dans le méthanol, ajoute de l'acide fumarique dans le méthanol et obtient 2,238 g du produit attendu F◊ ≃ 263αC avec décomposition.

### Exemple 4 : 4-/(2R-cis) 4-méthyl 6-/(méthylthio) méthyl/ 2-morpholinyl/ 1 H-indole (dl) et son fumarate neutre.

### Stade A :

### (2 RS, 6RS) 6-(1 H-indole 4-yl) 4-méthyl 2-morpholine paratoluène sulfonate méthanol.

On ajoute goutte à goutte à 10αC sous agitation et atmosphère inerte, une solution de 8,58 g de chlorure de tosyle dans 43 cm3 de pyridine à une solution de 5,542 g de base de l'exemple 3 dans 55 cm3 de pyridine, agite 3 H à 10αC et laisse au repos à 53◊C pendant 65 H environ. On verse alors dans 500 cm3 d'eau glacée, extrait au chlorure de méthylène, lave à l'aide d'une solution aqueuse de bicarbonate de sodium, sèche, évapore à sec sous pression réduite, chromatographie sur silice (Eluant : chlorure de méth ylène-acétone 1-1), et obtient 7,7 g du produit attendu.

### Stade B :

### 4-/(2R-cis) 4-méthyl 6-/(méthylthio) méthyl/ 2-morpholinyl/ 1 H-indole (dl) et son fumarate neutre

On ajoute à 03◊C sous agitation et atmosphère inerte -25 cm3 du diméthyl acétamide à 9 cm3 de méthyl mercaptan, ajoute lentement 1,8 g d'hydrure de sodium à 55 % dans l'huile, agite 1 H 30 mn, ajoute 2,5 g de produit obtenu au stade précédent en solution dans 15 cm3 de diméthyl acétamide et agite 2 H à température ambiante. On verse dans 400 cm3 d'eau glacée, extrait à l'acétate d'éthyle, lave à l'eau, et avec une solution aqueuse saturée en chlorure de sodium sèche, amène à sec, chromatographie sur silice (Eluant : chlorure de méthylène isopropanol 95-5), et obtient 1,165 g du produit attendu après recristallisation dans l'isopropanol. F◊ = 156◊ C.

### Formation du fumarate :

Par addition d'une solution d'acide fumarique dans le méthanol à une solution de 1,105 g de produit ci-dessus dissous dans l'isopropanol, on obtient 1,124 g du fumarate attendu. F◊ = 130◊C, puis 220◊C.

### Exemple 5 : (2 SR, 6 RS) 6-(1H-indol4-yl) 4-méthyl 2-morpholine acétonitrile et son fumarate neutre.

On ajoute 5 g de cyanure de sodium à une solution de 2 g de produit du stade A de l'exemple 4 dans 20 cm3 du diméthyl sulfoxyde et 5 cm3 d'eau, chauffe à 603◊C pendant 18 H. On verse dans l'eau le mélange réactionnel, extrait au chlorure de méthylène, lave à l'eau, sèche, amène à sec. On chromatographie le résidu sur silice (Eluant : chlorure de méthylène - méthanol 95/5) et obtient 1,24 g du produit attendu.

### Formation du fumarate :

On ajoute une solution de 332 mg d'acide fumarique dans 10 cm3 de méthanol à 5 cm3 d'une solution méthanolique de 1,32 g de produit obtenu comme ci-dessus, amène à sec, recristallise dans l'éthanol à 953◊C et obtient 1,40 g du produit attendu.

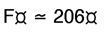

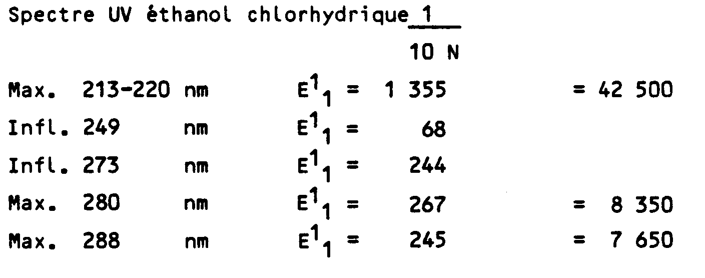

### Préparation du (2 RS, 6 RS) 6-(2,3-dihydro 1H-indol 4-yl) 4-méthyl 2-morpholine carboxylate d'éthyle.

### Stade 1 : N-/2-(1 H-indol 4-yl) 2-hydroxyéthyl/ carbamate de méthyle.

On ajoute goutte à goutte, sous agitation, une solution de 94 cm3 de chloroformiate de méthyle dans 285 cm3 d'acétate d'éthyle, à une suspension de 285 g de fumarate de l' a- (aminométhyl) 1 H-indole 4-méthanol et 270 g de bicarbonate de sodium dans 2 850 cm3 d'eau et 2 850 cm3 d'acétate d'éthyle. Après 1 H d'agitation, on décante, extrait à l'acétate d'éthyle la phase aqueuse, lave les phases organiques réunies à l'aide d'une solution aqueuse saturée en chlorure de sodium, sèche et évapore à sec. On dissout le produit obtenu dans du chlorure de méthylène, amène à sec, reprend le résidu dans 500 cm3 de méthanol chaud, laisse refroidir, ajoute de l'éther isopropylique, filtre l'insoluble, évapore le filtrat et obtient 202 g du produit attendu.

### Stade 2: N-/2-(2,3-dihydro 1 H-indol 4-yl) 2-hydroxyéthyl/ carbamate de méthyle.

On ajoute 35,5 g de complexe borane-triméthylamine, puis lentement et en refroidissant à 10αC 40,55 cm3 de solution aqueuse 12 N d'acide chlorhydrique, à une solution de 22,8 g de produit du stade précédent dans 570 cm3 de dioxanne, et agite pendant 16 heures à température ambiante. On verse dans 1 500 cm3 d'eau, lave à l'acétate d'éthyle, alcalinise à l'aide de 48 cm3 d'ammoniaque concentrée, extrait à l'acétate d'éthyle, sèche les phases organiques, filtre, évapore à sec, recristallise les 22,6 g de résidu obtenu dans le méthanol, et obti ent 13,125 g du produit attendu.
F◊≃ 174 ◊ C.

### Stade 3 : N-/2-(1-benzoyl 2,3-dihydro 1-H indol 4-yl) 2-hydroxyéthyl/ carbamate de méthyle.

On ajoute goutte à goutte, sous agitation et atmosphère inerte, en 10 mn, 66,6 cm3 de chlorure de benzoyle à une émulsion de 90,3 g de produit du stade précédent dans 2 000 cm3 de chloroforme et 573 cm3 de soude N. Après 2 H 30 mn d'agitation, on dilue par 11 d'eau, décante, extrait au chloroforme, lave à l'eau la phaseorganique, sèche, amène à sec sous pression réduite, cristallise dans l'acétate d'éthyle et obtient 113,5 g de produit attendu.
F◊ ≃ 143αC.

### Stade 4 : 2,3-dihydro a-/(méthylamino) méthyl/ 1-(phénylméthyl) 1 H-indol 4-méthanol.

On ajoute lentement, sous agitation et atmosphère inerte, une solution de 113 g de produit obtenu au stade précédent dans 1 130 cm3 de dioxanne à une suspension de 110 g d'hydrure d'aluminium-lithium et 55 g de chlorure d'aluminium dans 1 700 cm3 de dioxanne, après 1 H d'agitation entre 80αC et 50αC, refroidit à 10αC, détruit l'excès d'hydrure à l'aide d'un mélange dioxanne-eau-chlorure d'ammonium, dilue à l'eau, filtre, concentre le filtrat en éliminant environ 1 000 cm3 de solvant, ajoute de l'eau, extrait au chlorure de méthylène, lave à l'eau saturée au chlorure de sodium, sèche, évapore à sec et obtient 95 g du produit attendu brut. On agite le produit brut au reflux dans 11 d'acétate d'éthyle, filtre 8,5 g d'insoluble correspondant au chlorhydrate du produit attendu F = 179αC, concentre le filtrat à chaud à 400 cm3, laisse cristalliser et obtient 55,19 g de la base attendue F◊ 112◊C.

### Stade 5 : 3-//2-/2,3-dihydro 1-(phénylméthyl) 1 H-indol 4-yl/2-hydroxy éthyl/méthylamino/ 2-hydroxy propa- noate d'éthyle.

On porte au reflux pendant 4 heures sous agitation et atmosphère inerte 51,1 g de produit du stade précédent avec 511 cm3 d'éthanol 100◊ et 39 cm3 de glycidate d'éthyle, évapore à 40◊C sous pression réduite (20 mm, puis 0,5 mm Hg) et obtient 81,2 g de produit attendu impur que l'on utilise tel quel au stade suivant.

### Stade 6 : (2 RS, 6 SR) 6-/2,3-dihydro 1-(phénylméthyl) 1 H-indol 4-yl/ 4-méthyl 2-morpholine carboxylate d'éthyle.

On dissout 8,03 g de produit du stade précédent dans 70 cm3 de chlorure de méthylène, refroidit à -40◊C, ajoute sous atmosphère inerte 9,13 cm3 de N-chloro diisopropylamine, puis lentement, 11 cm3 de trisdiméthylaminophosphine. On agite 15 minutes à -40◊C, laisse remonter la température, dilue par 70 cm3 de chlorure de méthylène, lave à l'eau, puis à l'eau saturée en chlorure de sodium, sèche, amène à sec, chromatographie sur silice (ELuant : benzène-acétate d'éthyle 7-3 avec 0,5 % de triéthylamine) et obtient 4,706 g du produit attendu (Rf = 0,12).

### Stade 7 : (2 RS, 6RS) 6-(2,3-dihydro 1 H-indol 4-yl) 4-méthyl 2-morpholine carboxylate d'éthyle.

On dissout 4,7 g de produit du stade précédent dans 235 cm3 d'acide acétique, ajoute 1,18 g de palladium à 10 % sur charbon, hydrogène pendant 1 H, filtre, amène à sec, reprend dans 200 cm3 d'eau, alcalinise à pH 9 à l'aide d'ammoniaque concentrée, extrait au chlorure de méthylène, sèche, amène à sec et obtient 2,683 g du produit attendu après recristallisation dans l'éther isopropylique F◊ = 92◊ C

### Exemple 6 :

On a préparé des comprimés répondant à la formule :
- Fumarate neutre de 4-/(2 R-cis) 4-méthyl 6-/(méthylthio)méthyl/ 2-morpholinyl/ 1H-indole (dl) ................................ 10 mg ;
- Excipient q. s. pour un comprimé terminé à................. 100 mg• (Détail de L'excipient : lactose, amidon, talc, stéarate de magnésium).

### 1/. Test du labyrinthe en T après lésion partielle de la voie septo-hippocampique

### Principe :

On provoque, à l'aide d'une lésion électrolytique partielle de neurones de la voie septo-hippocampique, un disfonctionnement de la faculté cognitive chez des rats mâles ayant acquis un apprentissage dans un labyrinthe en forme de T, puis mesure la vitesse de récupération des facultés mnésiques perturbées.

On habitue les animaux à leur nouvel environnement les jours -9 à -6, repos les jours -5 et -4, on mène leur apprentissage les jours -3 à 0. A ce moment, la réponse des rats est posititive à 75-80 %. Au jour J 0, on effectue la lésion, repos les jours + 1 à + 3, et on contrôle les animaux au jour +4 ; à ce moment les réponses ne sont plus positives qu'à 50 %. Du jour + 5 à + 8, les rats sont testés après avoir été traités per os à l'aide de produits à tester 1 heure auparavant. Le jour + 11, les rats sont contrôlés sans traitement.

### Résultats :

On compare le pourcentage de réponses positives des animaux traités par rapport aux non-traités.

Le dérivé de l'exemple 4 améliore significativement la récupération des fonctions mnésiques à la dose de 20 mg/Kg ; il améliore donc les processus cognitifs.

### 2/. Test de l'énolase

Les cellules cérébrales en souffrance libèrent de l'énolase γγ, marqueur spécifique de lésions neuronales.

Les lésions sont réalisées chez la souris par injection sous-cutanée de 35 mg/Kg d'acide kaïnique. Le produit à tester est administré par voie intrapéritonéale à la dose de 1 mg/Kg 1 heure avant l'injection de l'acide kaïnique, neurotoxique. Un protecteur des cellules cérébrales en situation de souffrance diminue la concentration sérique d'énolase.

### Résultats :

Le produit de l'exemple 4 à la dose de 1 mg/Kg diminue de 34 % la concentration sérique d'énolase γγ. C'est donc un protecteur des cellules neuronales.

### 3/. Test d'activation neuronale

L'activation neuronale entraîne une augmentation du métabolisme correspondant se traduisant par une augmentation de la consommation de glucose, témoin de consommation d'énergie. On évalue cette augmentation en mesurant l'accumulation dans les cellules neuronales du 2-déoxy glucose 6-phosphate provenant de la transformation de 2-déoxy glucose ¹⁴ C, analogue du glucose administré à dose de traceur qui emprunte les mêmes voies d'entrée dans la cellule que le glucose, par l'hexokinase.

On observe que le produit de l'exemple 4 entraîne une augmentation de l'accumulation de 2- déoxyglucose 6-P dans l'hippocampe de rat. Il active donc sélectivement le métabolisme de certaines structures mésolimbiques.

### 4/. Etude de la toxicité aiguë

On a évalué les doses létales DLₒ des différents composés testés après administration par voie orale chez la souris.

On appelle DLₒ la dose maximale ne provoquant aucune mortalité en 8 jours.

Les résultats obtenus sont les suivants :

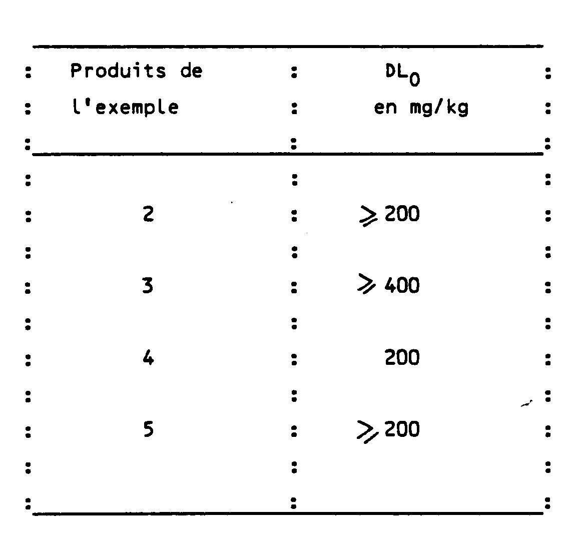

## Revendications (Revendications pour l'(les) Etat(s) contractant(s) suivant(s) : suivants : BE, CH, DE, GB, IT, LI, LU, NL, SE)

1. Les dérivés du 4-morpholinyl 1 H-indole, ainsi que leurs sels d'addition avec les acides minéraux ou organiques caractérisés en ce qu'ils répondent à la formule générale (1) : 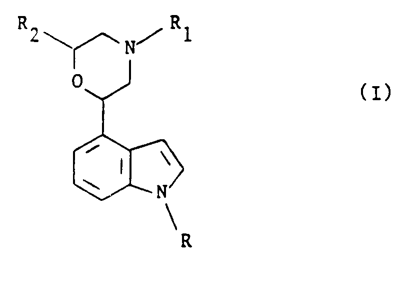 dans laquelle R représente un atome d'hydrogène ou un radical alkyle renfermant de 1 à 4 atomes de carbone, R₁ représente un atome d'hydrogène, un radical alkyle renfermant de 1 à 4 atomes de carbone, R₂ représente un radical hydroxyméthyle, alkylthiométhyle dont l'alkyle comprend de 1 à 3 atomes de carbone, cyanométhyle ou carboxy éventuellement estérifié par un alcool aliphatique renfermant de 1 à 5 atomes de carbone, ou amidifié par une amine de formule 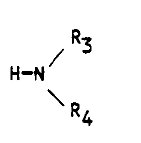 dans laquelle R₃ représente un atome d'hydrogène ou un radical alkyle renfermant de 1 à 4 atomes de carbone, et R₄ représente un radical alcoyle renfermant de 1 à 4 atomes de carbone.

2. Les dérivés du 4-morpholinyl 1 H-indole, ainsi que leurs sels d'addition avec les acides minéraux ou organiques, caractérisés en ce qu'ils répondent à la formule (I) de la revendicat ion 1, dans laquelle R représente un atome d'hydrogène.

3. Les dérivés du 4-morpholinyl 1 H-indole ainsi que leurs sels d'addition avec les acides minéraux ou organiques, caractérisés en ce qu'ils répondent à la formule (I) de la revendication 2, dans laquelle R₂ représente un radical hydroxyméthyle, alkyl thiométhyle ou cyano-méthyle ou un radical carboxy éventuellement amidifié par une amine de formule 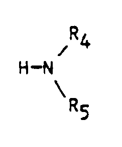 dans laquelle R₄ et R₅ représentent un radical alcoyle linéaire renfermant de 1 à 4 atomes de carbone.

4. Les dérivés du 4-morpholinyl 1 H-indole selon la revendication 3, ainsi que leurs sels d'addition avec les acides minéraux ou organiques, caractérisés en ce que R₂ représente un radical alkyltiométhyle dont l'alkyle comprend de 1 à 3 atomes de carbone.

5. le 4-/(2R-cis) 4-méthyl 6-/(méthylthio) méthyl/ 2-morpholinyl/ 1 H-indole, ainsi que ses sels d'addition avec les acides minéraux ou organiques.

6. Procédé de préparation des dérivés du 4-morpholinyl 1 H-indole tels que définis par la formule 1 de la revendication 1, ainsi que de leurs sels d'addition avec les acides minéraux ou organiques, caractérisé en ce que l'on soumet à une déshydrogénation un dérivé de formule (II) : 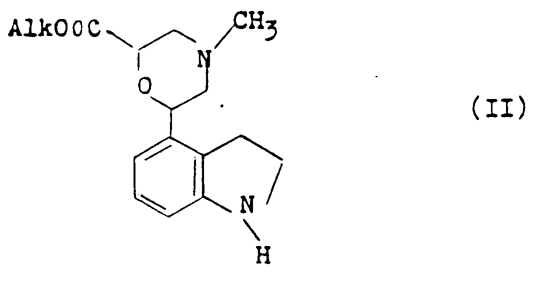 dans laquelle Alk représente un radical alkyle renfermant de 1 à 4 atomes de carbone, pour obtenir un dérivé de formule (I_{A}) : 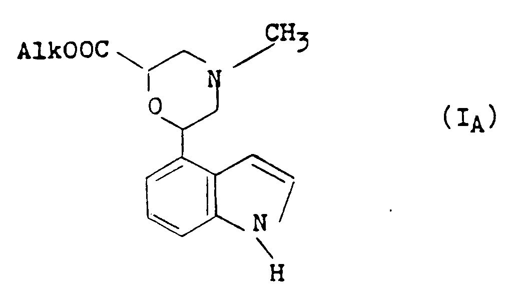 dans laquelle Alk a la signification déjà indiquée, que
- soit l'on isole et si désiré, salifie ;
- soit l'on déméthyle pour obtenir le produit de formule (I_{B}) : 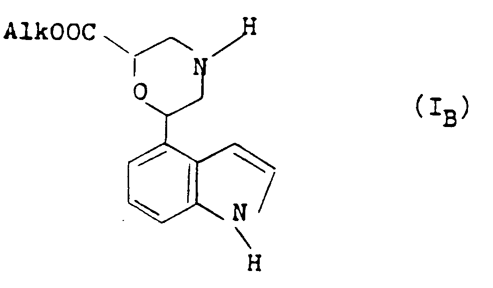 dans laquelle Alk a la signification déjà indiquée, que
- soit l'on isole et si désiré, salifie ;
- soit l'on fait réagir avec un agent d'alkylation, pour obtenir un produit de formule (l_{c}) : 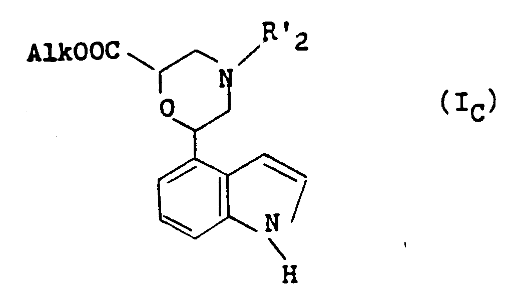 dans laquelle R'₂ a la signification de R₂ déjà indiquée à la revendication 1, à l'exception de l'hydrogène et alk a la signification déjà indiquée, que ou bien l'on isole, et, si désiré, salifie, ou bien l'on fait réagir avec un amidure alcalin, puis avec un halogénure d'alkyle de formule (IV) : 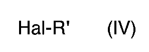 dans laquelle Hal représente un atome de chlore, de brome ou d'iode et R' représente un radical alkyle renfermant de 1 à 4 atomes de carbone, pour obtenir un produit de formule (I_{D}) : 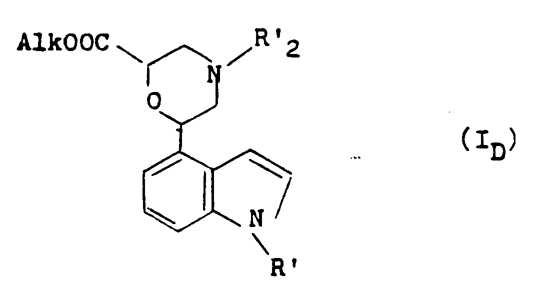 dans laquelle Alk, R' et R'₂ ont la signification déjà indiquée, que l'on isole et, si désiré, salifie ;
- soit l'on saponifie ledit produit de formule (I_{A}) pour obtenir un produit de formule (I_{E}) : 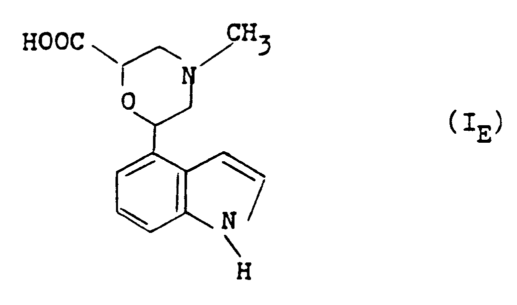 que ou bien l'on isole et, si désiré, salifie, ou bien l'on fait réagir avec une amine de formule (V) : 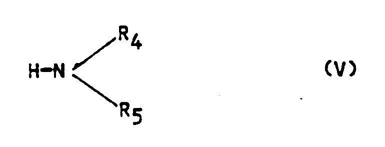 dans laquelle R4 et R5 ont la signification déjà indiquée à la revendication 3, pour obtenir un produit de formule (l_{F}) 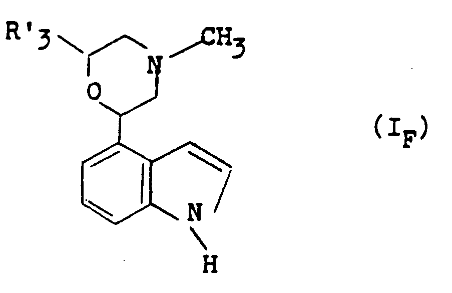 dans laquelle R'₃ représente un radical carboxy amidifié par l'amide de formule (V) définie ci-dessus, que l'on isole et si désiré, salifie ;
soit réduit ledit produit de formule (I_{A}) pour obtenir un produit de formule (I_{G}) : 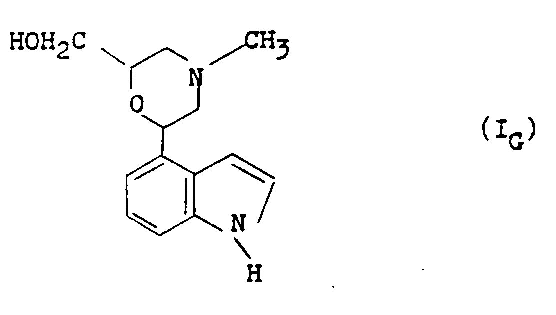 que ou bien l'on isole et, si désiré, salifie, ou bien l'on fait réagir avec le chlorure de méthane ou de paratoluène sulfonyle, pour obtenir un produit de formule (VI) : 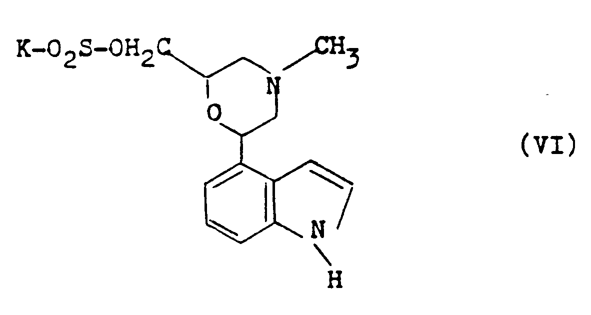 dans laquelle K représente un radical méthyle ou p-tolyle, que l'on fait réagir avec un alkylmercaptan ou avec un cyanure alcalin, pour obtenir un produit de formule (I_{H}) : 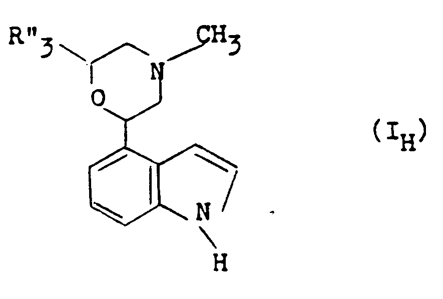 dans laquelle R"₃ représente un radical alkylthiométhyle ou cyanométhyle, que l'on isole et si désiré, salifie, puis effectue ensuite si désiré, sur les produits de formules (I_{E}), (I_{F}),(I_{G}) et (I_{H}), une ou plusieurs des réactions déjà indiquées ci-dessus pour les produits de formule (I_{A}), pour obtenir les produits correspondants entrant dans la formule (1), puis isole, et si désiré, salifie les produits ainsi obtenus.

7. Médicaments, caractérisés en ce qu'ils sont constitués par les nouveaux dérivés du 4-morpholinyl 1 H-indole, tels que définis par la formule (I) de la revendication 1, ainsi que par leurs sels d'addition avec les acides pharmaceutiquement acceptables.

8. Médicaments, caractérisés en ce qu'ils sont constitués par les nouveaux dérivés du 4-morpholinyl 1 H-indole, tels que définis à la revendication 2, ainsi que par leurs sels d'addition avec les acides pharmaceutiquement acceptables.

9. Médicaments, caractérisés en ce qu'ils sont constitués par les nouveaux dérivés du 4-morpholinyl 1 H-indole, tels que définis à la revendication 3 ou 4, ainsi que par leurs sels d'addition avec les acides pharmaceutiquement acceptables.

10. Médicaments, caractérisés en ce qu'ils sont constitués par le dérivé du 4-morpholinyl 1 H-indole tel que défini à la revendication 5, ainsi que par ses sels d'addition avec les acides pharmaceutiquement acceptables.

11. Compositions pharmaceutiques, caractérisées en ce qu'elles renferment, à titre de principe actif, l'un au moins des médicaments, tels que définis à l'une des revendications 7 à 10.

12. Les dérivés de formule (XIII) : 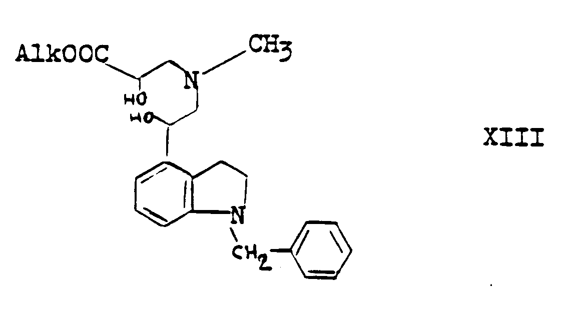 dans laquelle Alk représente un radical alkyle renfermant de 1 à 4 atomes de carbone.

13. Les dérivés de formule (XIV) : 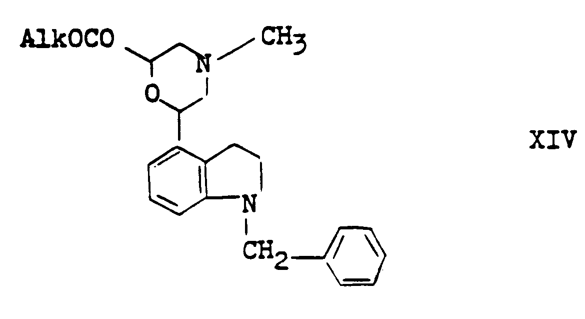 dans laquelle Alc représente un radical alkyle renfermant de 1 à 4 atomes de carbone.

## Revendications (Revendications pour l'(les) Etat(s) contractant(s) suivant(s) : suivant : AT)

1. Procédé de préparation des dérivés du 4-morpholinyl 1 H-indole, ainsi que de leurs sels d'addition avec les acides minéraux ou organiques, répondant à la formule générale (1) : 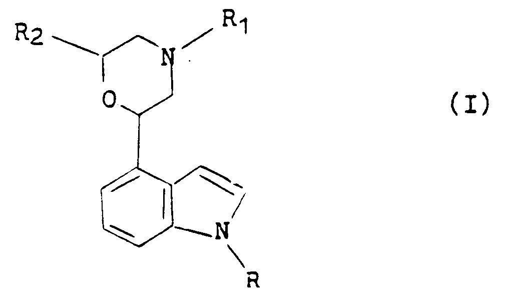 dans laquelle R représente un atome d'hydrogène ou un radical alkyle renfermant de 1 à 4 atomes de carbone, R₁ représente un atome d'hydrogène, un radical alkyle renfermant de 1 à 4 atomes de carbone, R₂ représente un radical hydroxyméthyle, alkylthiométhyle dont l'alkyle comprend de 1 à 3 atomes de carbone, cyanométhyle ou carboxy éventuellement estérifié par un alcool aliphatique renfermant de 1 à 5 atomes de carbone, ou amidifié par une amine de formule 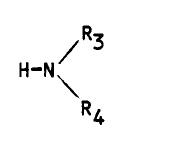 dans laquelle R₃ représente un atome d'hydrogène ou un radical alkyle renfermant de 1 à 4 atomes de carbone, et R₄ représente un radical alcoyle renfermant de 1 à 4 atomes de carbone, caractérisé en ce que l'on soumet à une déshydrogénation un dérivé de formule (II) : 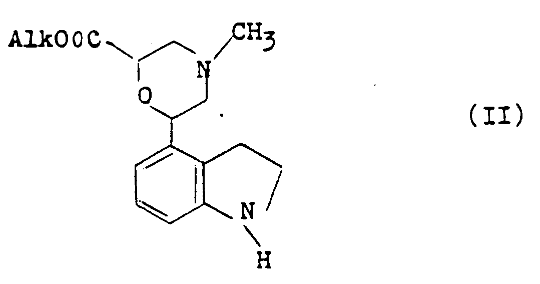 dans laquelle Alk représente un radical alkyle renfermant de 1 à 4 atomes de carbone, pour obtenir un dérivé de formule (I_{A}) : 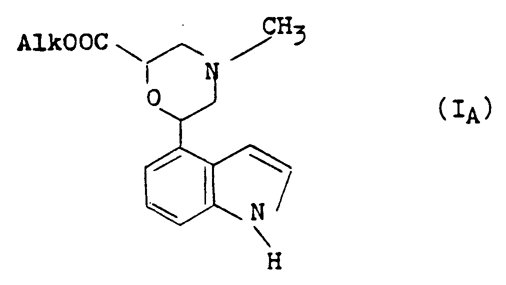 dans laquelle Alk a la signification déjà indiquée, que
- soit l'on isole et si désiré, salifie ;
- soit l'on déméthyle pour obtenir le produit de formule (I_{B}) : 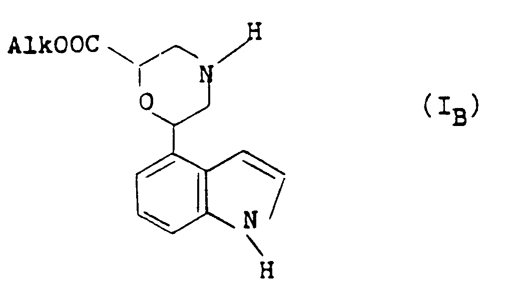 dans laquelle Alk a la signification déjà indiquée, que
- soit l'on isole et si désiré, salifie ;
- soit l'on fait réagir avec un agent d'alkylation, pour obtenir un produit de formule (l_{c}) : 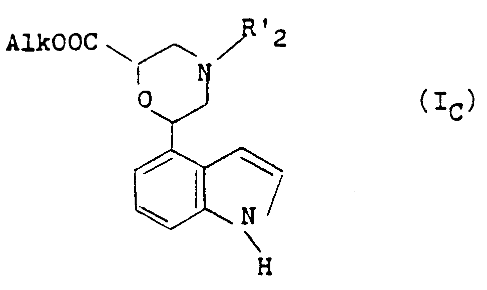 dans laquelle R'₂ a la signification de R₂ déjà indiquée à l'exception de l'hydrogène et alk a la signification déjà indiquée, que ou bien l'on isole, et, si désiré, salifie, ou bien l'on fait réagir avec un amidure alcalin, puis avec un halogénure d'alkyle de formule (IV) : 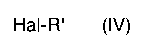 dans laquelle Hal représente un atome de chlore, de brome ou d'iode et R' représente un radical alkyle renfermant de 1 à 4 atomes de carbone, pour obtenir un produit de formule (I_{D}) : 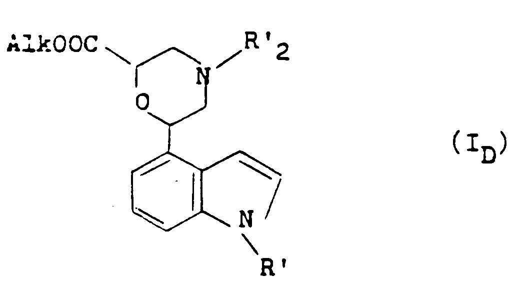 dans laquelle Alk, R' et R'₂ ont la signification déjà indiquée, que l'on isole et, si désiré, salifie ;
- soit l'on saponifie ledit produit de formule (I_{A}) pour obtenir un produit de formule (I_{E}) : 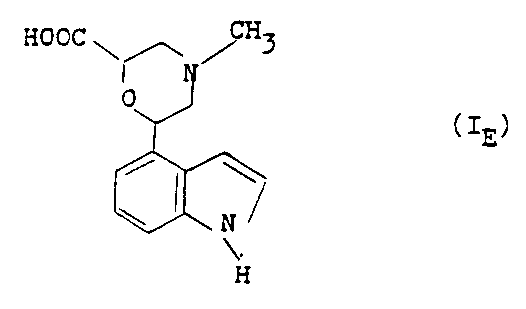 que ou bien l'on isole et, si désiré, salifie, ou bien l'on fait réagir avec une amine de formule (V) : 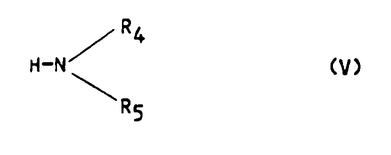 dans laquelle R₄ et R₅ représentent un radical alcoyle linéaire renfermant de 1 à 4 atomes de carbone, pour obtenir un produit de formule (I_{F}) 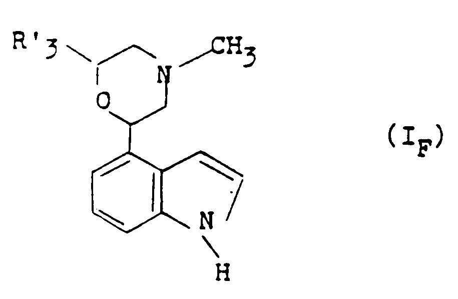 dans laquelle R'₃ représente un radical carboxy amidifié par l'amide de formule (V) définie ci-dessus, que l'on isole et si désiré, salifie ;
soit réduit ledit produit de formule (I_{A}) pour obtenir un produit de formule (I_{G}) : 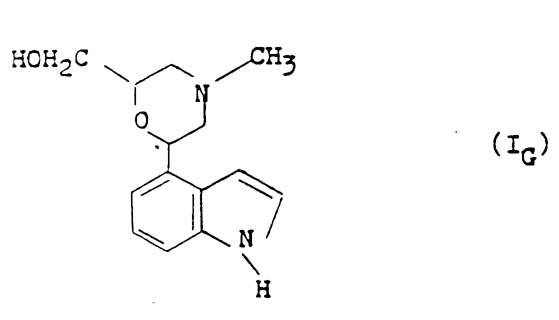 que ou bien l'on isole et, si désiré, salifie, ou bien l'on fait réagir avec le chlorure de méthane ou de paratoluène sulfonyle, pour obtenir un produit de formule (VI) : 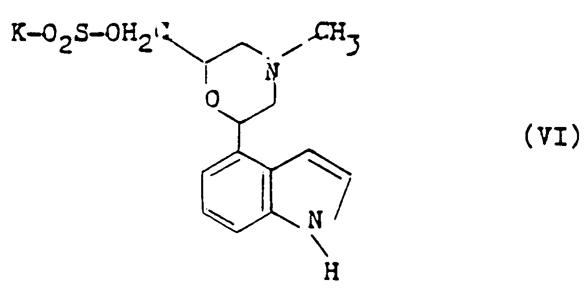 dans laquelle K représente un radical méthyle ou p-tolyle, que l'on fait réagir avec un alkylmercaptan ou avec un cyanure alcalin, pour obtenir un produit de formule (I_{H}) : 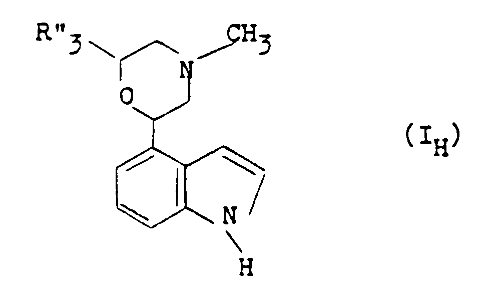 dans laquelle R"₃ représente un radical alcoylthiométhyle ou cyanométhyle, que l'on isole et si désiré, salifie, puis effectue ensuite si désiré, sur les produits de formules (I_{E}), (I_{F}),(I_{G}) et (I_{H}), une ou plusieurs des réactions déjà indiquées ci-dessus pour les produits de formule (I_{A}), pour obtenir les produits correspondants entrant dans la formule (1), puis isole, et si désiré, salifie les produits ainsi obtenus.

2. Procédé selon la revendication 1, caractérisé en ce que l'on prépare des dérivés répondant à la formule (I) de la revendication 1, dans laquelle R représente un atome d'hydrogène, ainsi que leurs sels d'addition avec les acides minéraux ou organiques.

3. Procédé selon la revendication 1 ou 2, caractérisé en ce que l'on prépare des dérivés répondant à la formule (1), dans laquelle R₂ représente un radical hydroxyméthyle, alkyl thiométhyle dont l'alkyle comprend de 1 à 3 atomes de carbone ou cyano-méthyle ou un radical carboxy éventuellement amidifié par une amine de formule 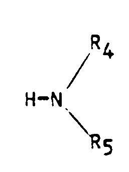 dans laquelle R₄ et R₅ représentent un radical alcoyle linéaire renfermant de 1 à 4 atomes de carbone, ainsi que leurs sels d'addition aavec les acides minéraux ou organiques.

4. Procédé selon la revendication 3, caractérisé en ce que l'on prépare des dérivés répondant à la formule (1), dans laquelle R₂ représente un radical alkylthiométhyle dont l'alkyle comprend de 1 à 3 atomes de carbone, ainsi que leurs sels d'addition avec les acides minéraux ou organiques.

5. Procédé selon l'une quelconque des revendications 1 à 4, caractérisé 5 en ce que l'on prépare le 4-/(2R-cis) 4-méthyl 6-/(méthylthio) méthyl/ 2-morpholinyl/ 1 H-indole, ainsi que ses sels d'addition avec les acides minéraux ou organiques.

## Revendications (Revendications pour l'(les) Etat(s) contractant(s) suivant(s) : suivant : ES)

1. Procédé de préparation des dérivés du 4-morpholinyl 1 H-indole, ainsi que de leurs sels d'addition avec les acides minéraux ou organiques, répondant à la formule générale (1) : 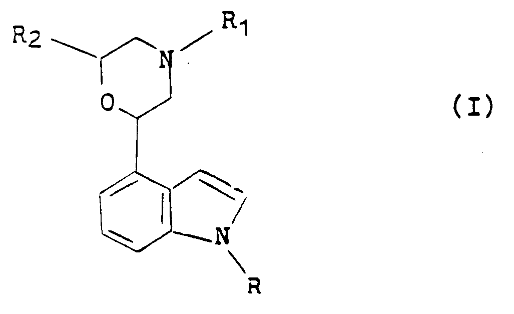 dans laquelle R représente un atome d'hydrogène ou un radical alkyle renfermant de 1 à 4 atomes de carbone, R₁ représente un atome d'hydrogène, un radical alkyle renfermant de 1 à 4 atomes de carbone, R₂ représente un radical hydroxyméthyle, alkylthiométhyle dont l'alkyle comprend de 1 à 3 atomes de carbone, cyanométhyle ou carboxy éventuellement estérifié par un alcool aliphatique renfermant de 1 à 5 atomes de carbone, ou amidifié par une amine de formule 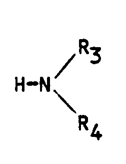 dans laquelle R₃ représente un atome d'hydrogène ou un radical alkyle renfermant de 1 à 4 atomes de carbone, et R₄ représente un radical alcoyle renfermant de 1 à 4 atomes de carbone, caractérisé en ce que l'on soumet à une déshydrogénation un dérivé de formule (II) : 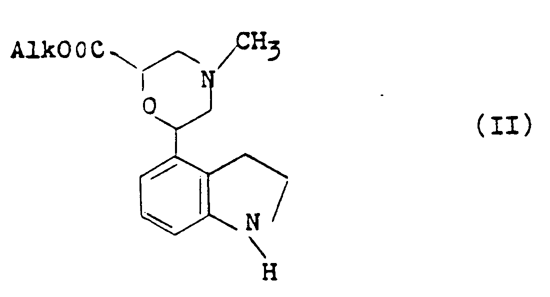 dans laquelle Alk représente un radical alkyle renfermant de 1 à 4 atomes de carbone, pour obtenir un dérivé de formule (I_{A}) : 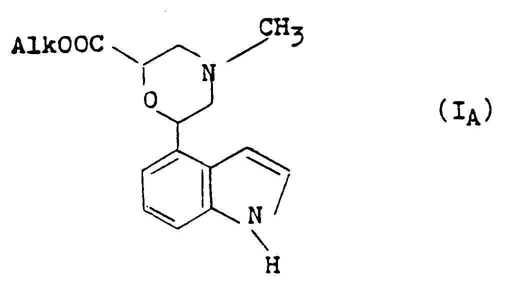 dans laquelle Alk a la signification déjà indiquée, que
- soit l'on isole et si désiré, salifie ;
- soit l'on déméthyle pour obtenir le produit de formule (I_{B}) : 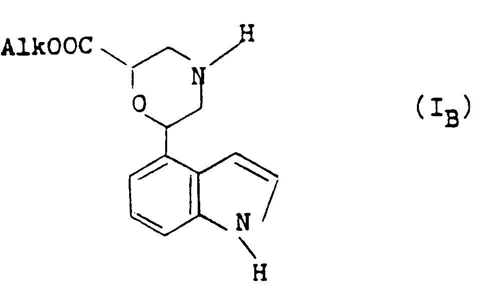 dans laquelle Alk a la signification déjà indiquée, que
- soit l'on isole et si désiré, salifie ;
- soit l'on fait réagir avec un agent d'alkylation, pour obtenir un produit de formule (l_{c}) : 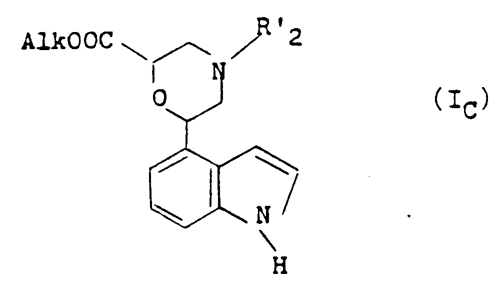 dans laquelle R'₂ a la signification de R₂ déjà indiquée à l'exception de l'hydrogène et alk a la signification déjà indiquée, que ou bien l'on isole, et, si désiré, salifie, ou bien l'on fait réagir avec un amidure alcalin, puis avec un halogénure d'alkyle de formule (IV) : 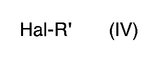 dans laquelle Hal représente un atome de chlore, de brome ou d'iode et R' représente un radical alkyle renfermant de 1 à 4 atomes de carbone, pour obtenir un produit de formule (I_{D}) : 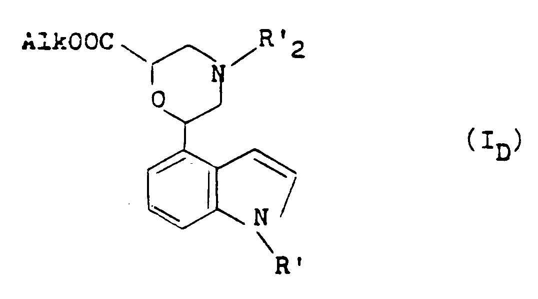 dans laquelle Alk, R' et R'₂ ont la signification déjà indiquée, que l'on isole et, si désiré, salifie ;
- soit l'on saponifie ledit produit de formule (I_{A}) pour obtenir un produit de formule (I_{E}) : 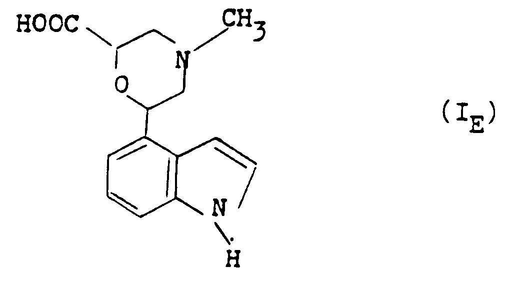 que ou bien l'on isole et, si désiré, salifie, ou bien l'on fait réagir avec une amine de formule (V) : 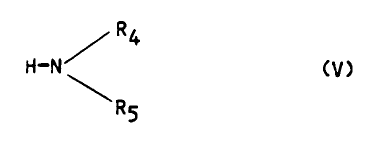 dans laquelle R₄ et R₅ représentent un radical alcoyle linéaire renfermant de 1 à 4 atomes de carbone, pour obtenir un produit de formule (I_{F}) 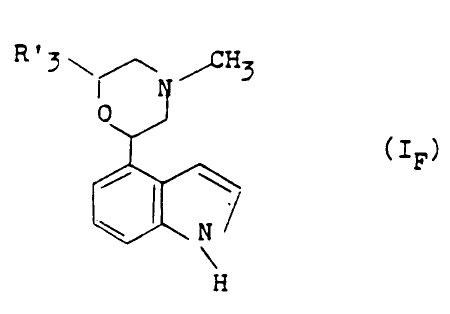 dans laquelle R'₃ représente un radical carboxy amidifié par l'amide de formule (V) définie ci-dessus, que l'on isole et si désiré, salifie ; soit réduit ledit produit de formule (I_{A}) pour obtenir un produit de formule (I_{G}) : 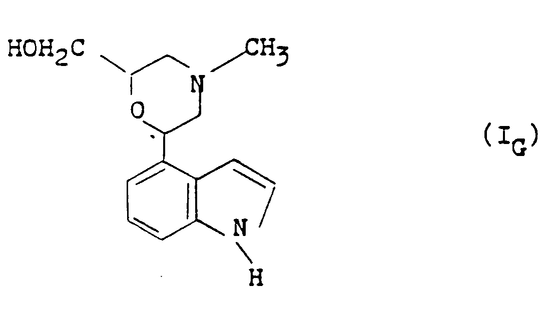 que ou bien l'on isole et, si désiré, salifie, ou bien l'on fait réagir avec le chlorure de méthane ou de paratoluène sulfonyle, pour obtenir un produit de formule (VI) : 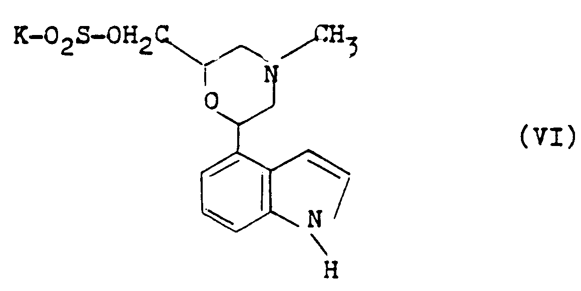 dans laquelle K représente un radical méthyle ou p-tolyle, que l'on fait réagir avec un alkylmercaptan ou avec un cyanure alcalin, pour obtenir un produit de formule (I_{H}) : 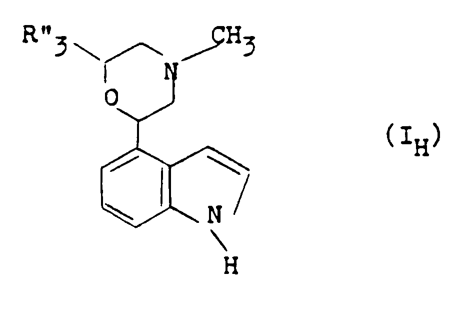 dans laquelle R"₃ représente un radical alcoylthiométhyle ou cyanométhyle, que l'on isole et si désiré, salifie, puis effectue ensuite si désiré, sur les produits de formules (I_{E}), (I_{F}),(I_{G}) et (I_{H}), une ou plusieurs des réactions déjà indiquées ci-dessus pour les produits de formule (I_{A}), pour obtenir les produits correspondants entrant dans la formule (1), puis isole, et si désiré, salifie les produits ainsi obtenus.

2. Procédé selon la revendication 1, caractérisé en ce que l'on prépare des dérivés répondant à la formule (I) de la revendication 1, dans laquelle R représente un atome d'hydrogène, ainsi que leurs sels d'addition avec les acides minéraux ou organiques.

3. Procédé selon la revendication 1 ou 2, caractérisé en ce que l'on prépare des dérivés répondant à la formule (1), dans laquelle R₂ représente un radical hydroxyméthyle, alkyl thiométhyle dont l'alkyle comprend de 1 à 3 atomes de carbone ou cyano-méthyle ou un radical carboxy éventuellement amidifié par une amine de formule 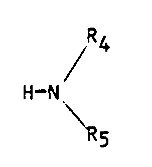 dans laquelle R₄ et R₅ représentent un radical alcoyle linéaire renfermant de 1 à 4 atomes de carbone, ainsi que leurs sels d'addition aavec les acides minéraux ou organiques.

4. Procédé selon la revendication 3, caractérisé en ce que l'on prépare des dérivés répondant à la formule (1), dans laquelle R₂ représente un radical alkylthiométhyle dont l'alkyle comprend de 1 à 3 atomes de carbone, ainsi que leurs sels d'addition avec les acides minéraux ou organiques.

5. Procédé selon l'une quelconque des revendications 1 à 4, caractérisé en ce que l'on prépare le 4-/(2R-cis) 4-méthyl 6-/(méthylthio) méthyl/ 2-morpholinyl/ 1 H-indole, ainsi que ses sels d'addition avec les acides minéraux ou organiques.

## Revendications (Revendications pour l'(les) Etat(s) contractant(s) suivant(s) : suivant : GR)

1. Procédé de préparation des dérivés du 4-morpholinyl 1 H-indole, ainsi que de leurs sels d'addition avec les acides minéraux ou organiques, répondant à la formule générale (1) : 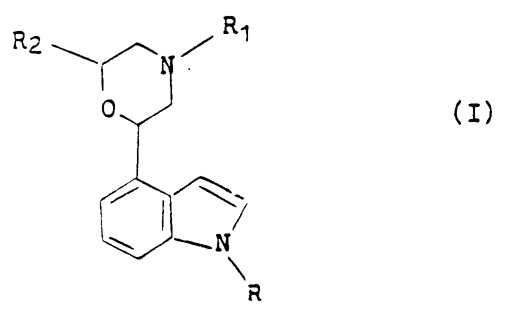 dans laquelle R représente un atome d'hydrogène ou un radical alkyle renfermant de 1 à 4 atomes de carbone, R₁ représente un atome d'hydrogène, un radical alkyle renfermant de 1 à 4 atomes de carbone, R₂ représente un radical hydroxyméthyle, alkylthiométhyle dont l'alkyle comprend de 1 à 3 atomes de carbone, cyanométhyle ou carboxy éventuellement estérifié par un alcool aliphatique renfermant de 1 à 5 atomes de carbone, ou amidifié par une amine de formule 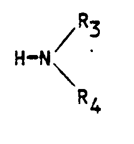 dans laquelle R₃ représente un atome d'hydrogène ou un radical alkyle renfermant de 1 à 4 atomes de carbone, et R₄ représente un radical alcoyle renfermant de 1 à 4 atomes de carbone, caractérisé en ce que l'on soumet à une déshydrogénation un dérivé de formule (II) : 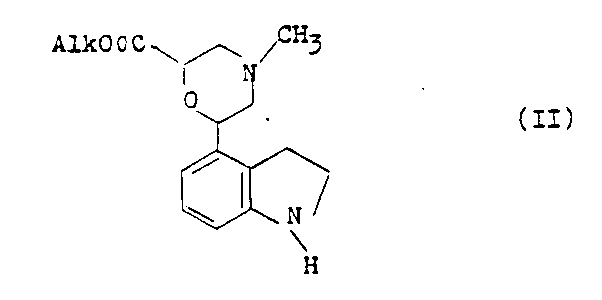 dans laquelle Alk représente un radical alkyle renfermant de 1 à 4 atomes de carbone, pour obtenir un dérivé de formule (I_{A}) : 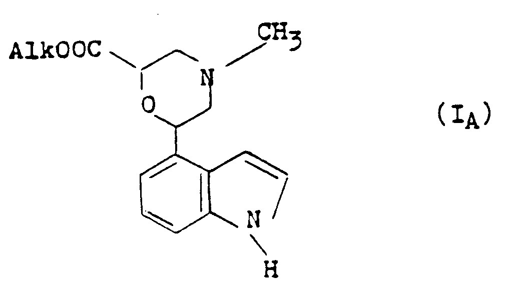 dans laquelle Alk a la signification déjà indiquée, que
- soit l'on isole et si désiré, salifie ;
- soit l'on déméthyle pour obtenir le produit de formule (I_{B}) : 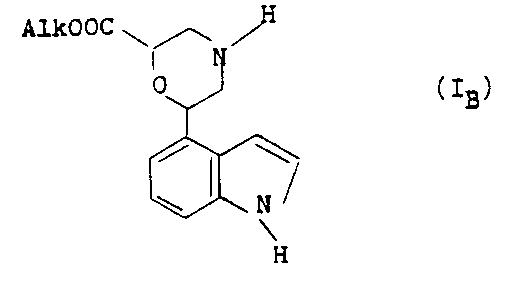 dans laquelle Alk a la signification déjà indiquée, que
- soit l'on isole et si désiré, salifie ;
- soit l'on fait réagir avec un agent d'alkylation, pour obtenir un produit de formule (l_{c}) : 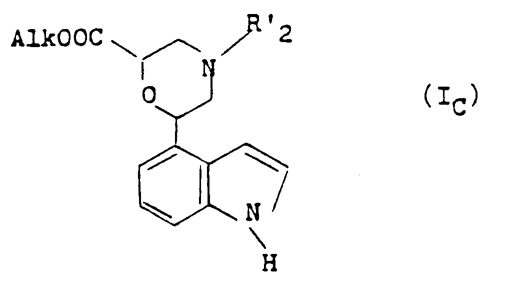 dans laquelle R'₂ a la signification de R₂ déjà indiquée à l'exception de l'hydrogène et alk a la signification déjà indiquée, que ou bien l'on isole, et, si désiré, salifie, ou bien l'on fait réagir avec un amidure alcalin, puis avec un halogénure d'alkyle de formule (IV) : 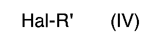 dans laquelle Hal représente un atome de chlore, de brome ou d'iode et R' représente un radical alkyle renfermant de 1 à 4 atomes de carbone, pour obtenir un produit de formule (I_{D}) : 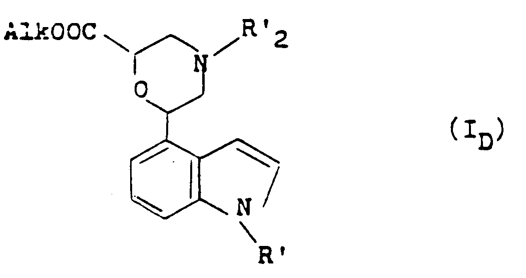 dans laquelle Alk, R' et R'₂ ont la signification déjà indiquée, que l'on isole et, si désiré, salifie ;
- soit l'on saponifie ledit produit de formule (I_{A}) pour obtenir un produit de formule (I_{E}) : 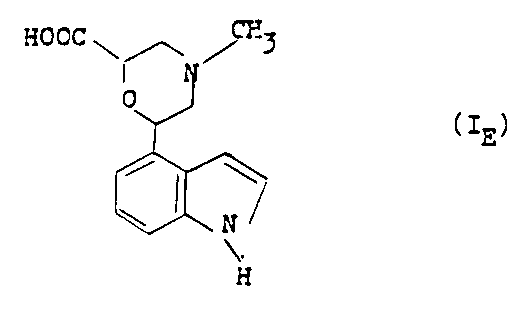 que ou bien l'on isole et, si désiré, salifie, ou bien l'on fait réagir avec une amine de formule (V) : 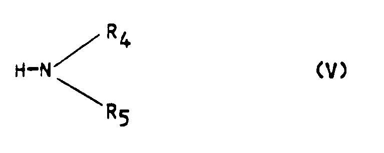 dans laquelle R₄ et R₅ représentent un radical alcoyle linéaire renfermant de 1 à 4 atomes de carbone, pour obtenir un produit de formule (I_{F}) 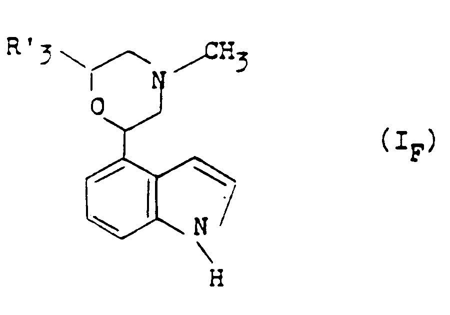 dans laquelle R'₃ représente un radical carboxy amidifié par l'amide de formule (V) définie ci-dessus, que l'on isole et si désiré, salifie ; soit réduit ledit produit de formule (I_{A}) pour obtenir un produit de formule (I_{G}) : 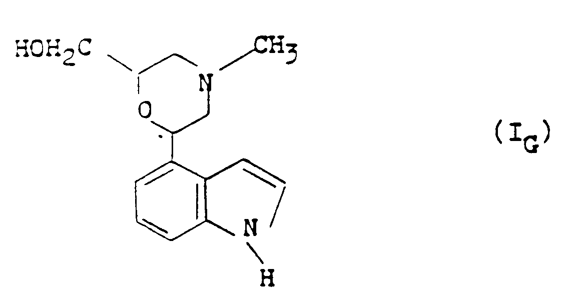 que ou bien l'on isole et, si désiré, salifie, ou bien l'on fait réagir avec le chlorure de méthane ou de paratoluène sulfonyle, pour obtenir un produit de formule (VI) : 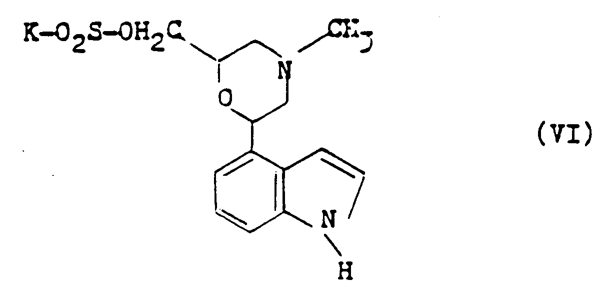 dans laquelle K représente un radical méthyle ou p-tolyle, que l'on fait réagir avec un alkylmercaptan ou avec un cyanure alcalin, pour obtenir un produit de formule (I_{H}) : 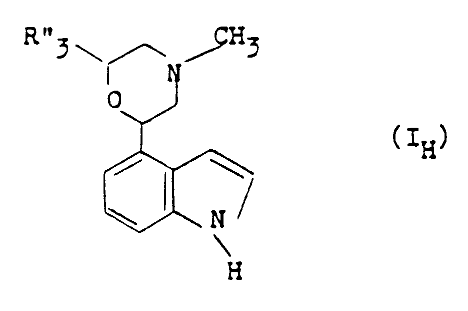 dans laquelle R"₃ représente un radical alcoylthiométhyle ou cyanométhyle, que l'on isole et si désiré, salifie, puis effectue ensuite si désiré, sur les produits de formules (I_{E}), (I_{F}),(I_{G}) et (I_{H}), une ou plusieurs des réactions déjà indiquées ci-dessus pour les produits de formule (I_{A}), pour obtenir les produits correspondants entrant dans la formule (1), puis isole, et si désiré, salifie les produits ainsi obtenus.

2. Procédé selon la revendication 1, caractérisé en ce que l'on prépare des dérivés répondant à la formule (I) de la revendication 1, dans laquelle R représente un atome d'hydrogène, ainsi que leurs sels d'addition avec les acides minéraux ou organiques.

3. Procédé selon la revendication 1 ou 2, caractérisé en ce que l'on prépare des dérivés répondant à la formule (1), dans laquelle R₂ représente un radical hydroxyméthyle, alkyl thiométhyle dont l'alkyle comprend de 1 à 3 atomes de carbone ou cyano-méthyle ou un radical carboxy éventuellement amidifié par une amine de formule 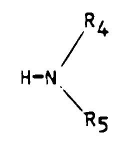 dans laquelle R₄ et R₅ représentent un radical alcoyle linéaire renfermant de 1 à 4 atomes de carbone, ainsi que leurs sels d'addition aavec les acides minéraux ou organiques.

4. Procédé selon la revendication 3, caractérisé en ce que l'on prépare des dérivés répondant à la formule (1), dans laquelle R₂ représente un radical alkylthiométhyle dont l'alkyle comprend de 1 à 3 atomes de carbone, ainsi que leurs sels d'addition avec les acides minéraux ou organiques.

5. Procédé selon l'une quelconque des revendications 1 à 4, caractérisé en ce que l'on prépare le 4-/(2R-cis) 4-méthyl 6-/(méthylthio) méthyl/ 2-morpholinyl/ 1 H-indole, ainsi que ses sels d'addition avec les acides minéraux ou organiques.

## Claims (Claims for the following Contracting State(s) : BE, CH, DE, GB, IT, LI, LU, NL, SE)

1. The derivatives of 4-morpholinyl-1 H-indole, as well as their addition salts with mineral or organic acids, characterized in that they correspond to the general formula (I): 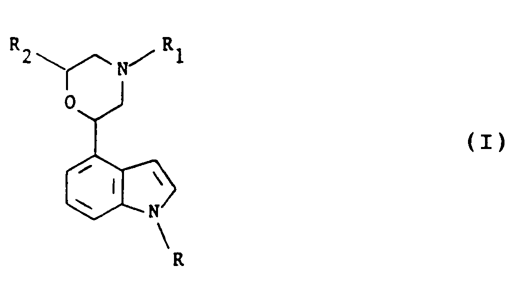 in which R represents a hydrogen atom or an alkyl radical containing 1 to 4 carbon atoms, R₁ represents a hydrogen atom, an alkyl radical containing 1 to 4 carbon atoms, R₂ represents a hydroxymethyl radical, an alkylthiomethyl radical the alkyl of which contains 1 to 3 carbon atoms, a cyanomethyl radical or a carboxy radical optionally esterified by an aliphatic alcohol containing 1 to 5 carbon atoms, or amidified by an amine of formula 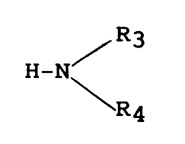 in which R₃ represents a hydrogen atom or an alkyl radical containing 1 to 4 carbon atoms, and R₄ represents an alkyl radical containing 1 to 4 carbon atoms.

2. The derivatives of 4-morpholinyl-1 H-indole, as well as their addition salts with mineral or organic acids, characterized in that they correspond to formula (I) of claim 1, in which R represents a hydrogen atom.

3. The derivatives of 4-morpholinyl-1 H-indole, as well as their addition salts with mineral or organic acids, characterized in that they correspond to formula (I) of claim 2, in which R₂ represents a hydroxymethyl radical, an alkylthiomethyl radical the alkyl of which contains 1 to 3 carbon atoms or a cyanomethyl radical or a carboxy radical optionally amidified by an amine of formula 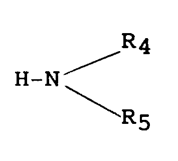 in which R₄ and R₅ represent a linear alkyl radical containing 1 to 4 carbon atoms.

4. The derivatives of 4-morpholinyl-1 H-indole according to claim 3, as well as their addition salts with mineral or organic acids, characterized in that R₂ represents an alkylthiomethyl radical the alkyl of which contains 1 to 3 carbon atoms.

5. 4-/(2R-cis)-4-methyl-6-/(methylthio)-methyl/-2-morpholinyl/-1 H-indol, as well as their addition salts with mineral or organic acids.

6. Preparation process for derivatives of 4-morpholinyl-1 H-indole as defined by formula (I) of claim 1, as well as their addition salts with mineral or organic acids, characterized in that a derivative of formula (II): 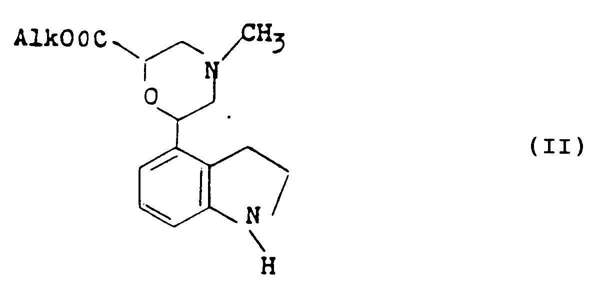 in which Alk represents an alkyl radical containing 1 to 4 carbon atoms, is subjected to a dehydrogenation in order to obtain a derivative of formula (I_{A}): 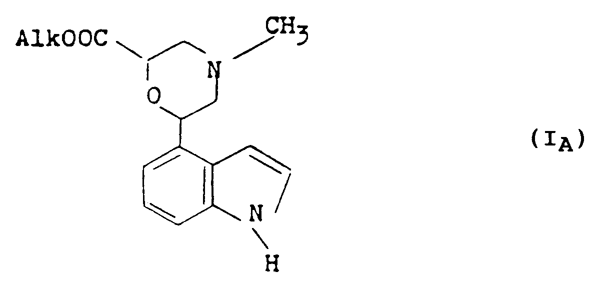 in which Alk has the meaning already indicated, which
- either is isolated and, if desired, salified;
- or is demethylated in order to obtain the product of formula (I_{B}): 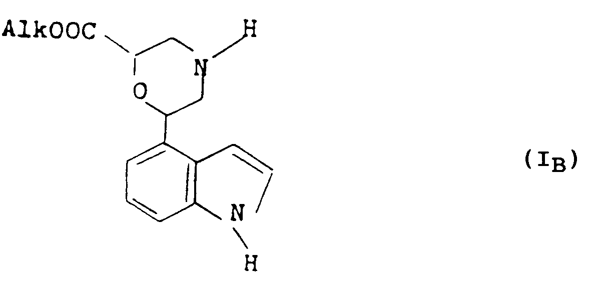 in which Alk has the meaning already indicated, which
- either is isolated and, if desired, salified;
- or is reacted with an alkylation agent in order to obtain a product of formula (Iₑ): 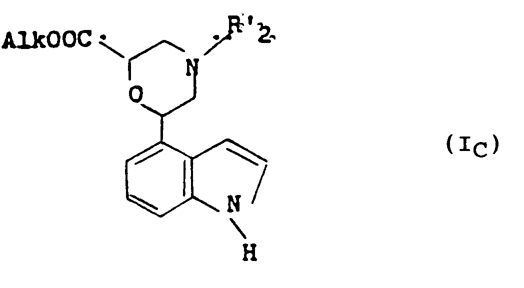 in which R'₂ has the meaning of R₂ already indicated in claim 1, with the exception of hydrogen, and Alk has the meaning already indicated, which either is isolated and, if desired, salified, or is reacted with an alkaline amide, then with an alkyl halide of formula (IV): 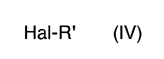 in which Hal represents a chlorine, bromine or iodine atom and R' represents an alkyl radical containing 1 to 4 carbon atoms, in order to obtain a product of formula (l_{D}): 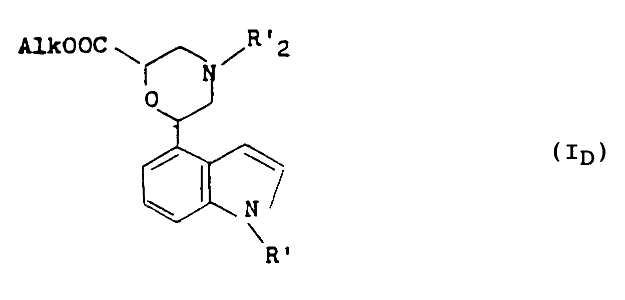 in which Alk, R' and R'₂ have the meaning already indicated, which is isolated and, if desired, salified;
- or the said product of formula (I_{A}) is saponified in order to obtain a product of formula (I_{E}): 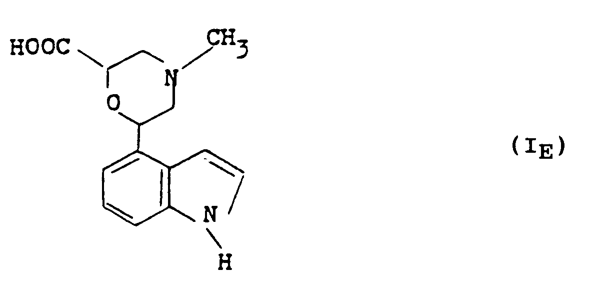 which either is isolated and, if desired, salified, or is reacted with an amine of formula (V): 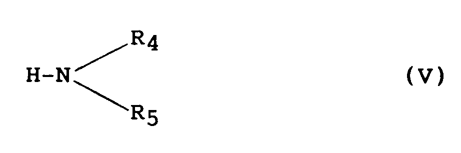 in which R₄ and R₅ have the meaning already indicated in claim 3, in order to obtain a product of formula (IF): 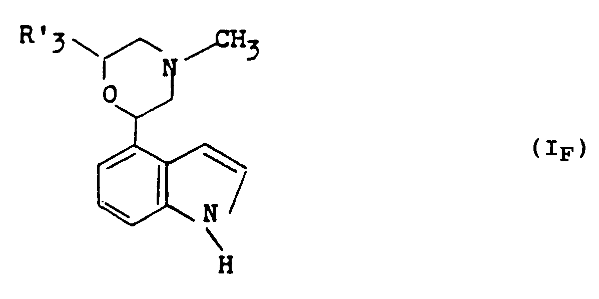 in which R'₃ represents a carboxy radical amidified by the amide of formula (V) defined above, which is isolated and if desired, salified; or the said product of formula (I_{A}) is reduced in order to obtain a product of formula (I_{G}): 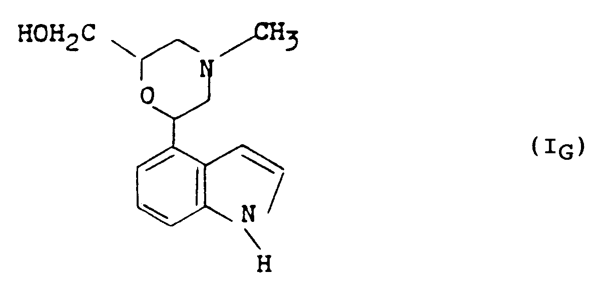 which either is isolated and, if desired, salified, or is reacted with methane chloride or paratoluene sulphonyl chloride, in order to obtain a product of formula (VI): 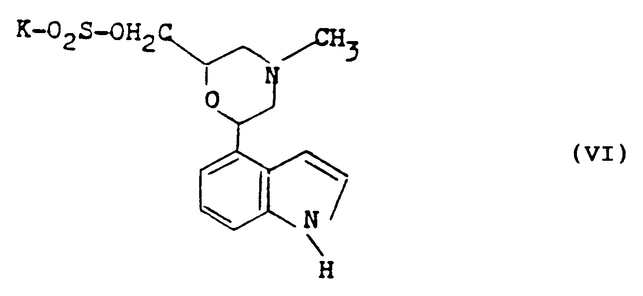 in which K represents a methyl or p-tolyl radical, which is reacted with an alkylmercaptan or with an alkaline cyanide, in order to obtain a product of formula (I_{H}): 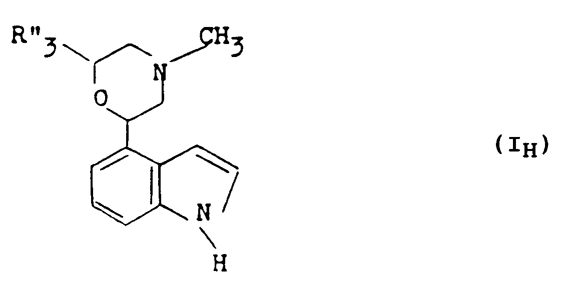 in which R"₃ represents an alkylthiomethyl or cyanomethyl radical, which is isolated and if desired, salified, then if desired one or more of the reactions already indicated above for the products of formula (I_{A}) are carried out on the products of formulae (I_{E}), (IF), (l_{G}) and (I_{H}), in order to obtain the corresponding products of formula (I), then the products thus obtained are isolated and if desired salified.

7. Medicaments, characterized in that they are constituted by new derivatives of 4-morpholinyl-1 H-indole, as defined by formula (I) of claim 1, as well as by their addition salts with pharmaceutically acceptable acids.

8. Medicaments, characterized in that they are constituted by new derivatives of 4-morpholinyl-1 H-indole, as defined in claim 2, as well as by their addition salts with pharmaceutically acceptable acids.

9. Medicaments, characterized in that they are constituted by new derivatives of 4-morpholinyl-1 H-indole, as defined in claim 3 or 4, as well as by their addition salts with pharmaceutically acceptable acids.

10. Medicaments, characterized in that they are constituted by the derivative of 4-morpholinyl-1 H-indole as defined in claim 5, as well as by their addition salts with pharmaceutically acceptable acids.

11. Pharmaceutical compositions, characterized in that they contain, as active ingredient, at least one of the medicaments as defined in one of claims 7 to 10.

12. The derivatives of formula (XIII): 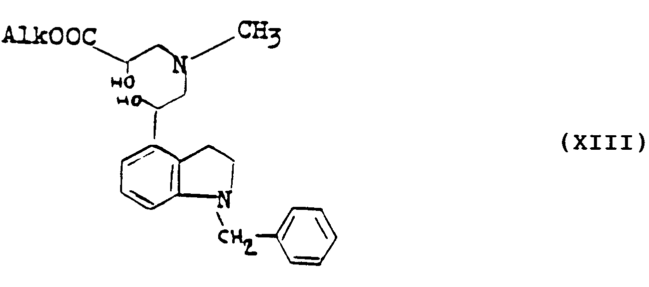 in which Alk represents an alkyl radical containing 1 to 4 carbon atoms.

13. The derivatives of formula (XIV): 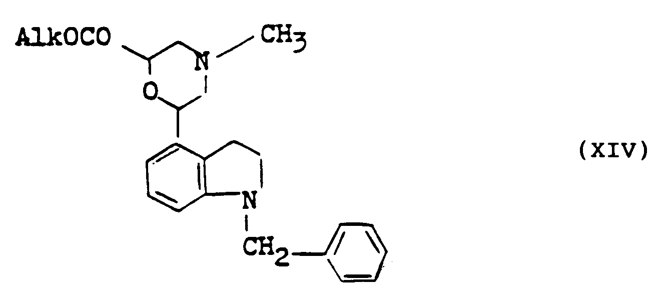 in which Alk represents an alkyl radical containing 1 to 4 carbon atoms.

## Claims (Claims for the following Contracting State(s) : AT)

1. Preparation process for derivatives of 4-morpholinyl-1 H-indole, as well as their addition salts with mineral or organic acids, corresponding to general formula (I): 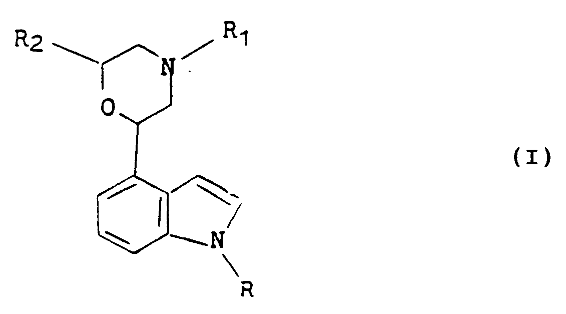 in which R represents a hydrogen atom or an alkyl radical containing 1 to 4 carbon atoms, R₁ represents a hydrogen atom, an alkyl radical containing 1 to 4 carbon atoms, R₂ represents a hydroxymethyl radical or an alkylthiomethyl radical the alkyl of which contains 1 to 3 carbon atoms, a cyanomethyl radical or a carboxy radical optionally esterified by an aliphatic alcohol containing 1 to 5 carbon atoms, or amidified by an amine of formula 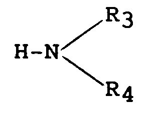 in which R₃ represents a hydrogen atom or an alkyl radical containing 1 to 4 carbon atoms, and R₄ represents an alkyl radical containing 1 to 4 carbon atoms, characterized in that a derivative of formula (II): 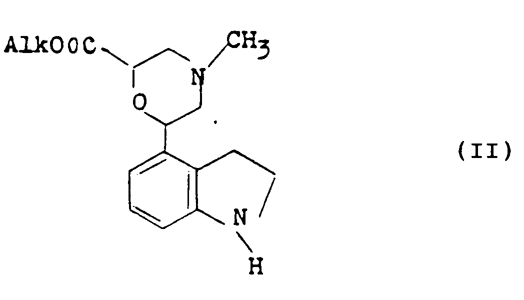 in which Alk represents an alkyl radical containing 1 to 4 carbon atoms, is subjected to a dehydrogenation in order to obtain a derivative of formula (I_{A}): 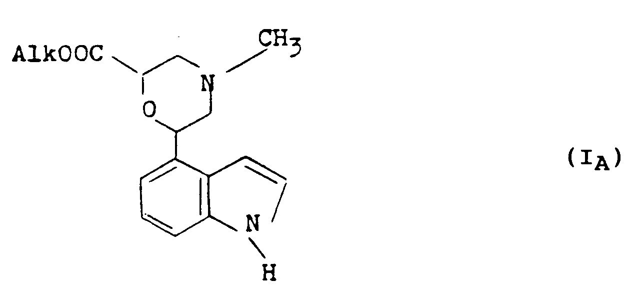 in which Alk has the meaning already indicated, which
- either is isolated and if desired, salified;
- or is demethylated in order to obtain the product of formula (I_{B}): 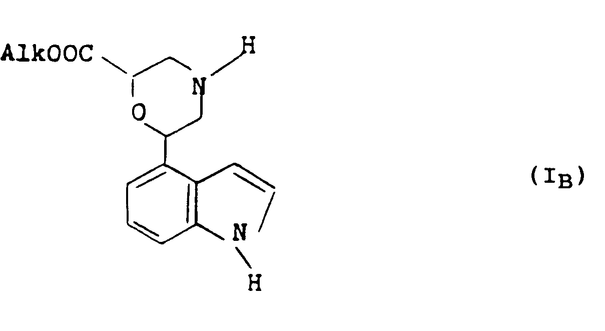 in which Alk has the meaning already indicated, which
- either is isolated and, if desired, salified;
- or is reacted with an alkylation agent, in order to obtain a product of formula (Iₑ): 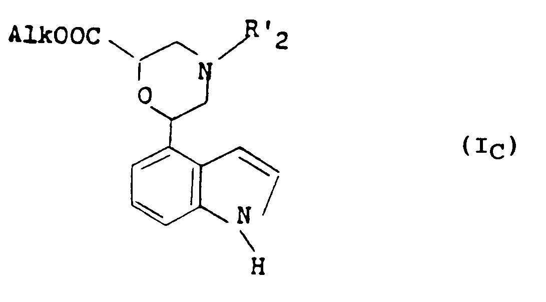 in which R'₂ has the meaning of R₂ already indicated with the exception of hydrogen and Alk has the meaning already indicated, which either is isolated and, if desired, salified, or is reacted with an alkaline amide, then with an alkyl halide of formula (IV): 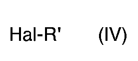 in which Hal represents a chlorine, bromine or iodine atom and R' represents an alkyl radical containing 1 to 4 carbon atoms, in order to obtain a product of formula (l_{D}): 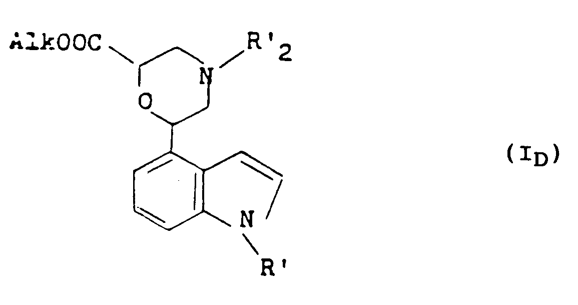 in which Alk, R' and R'₂ have the meaning already indicated, which is isolated and, if desired, salified;
- or the said product of formula (I_{A}) is saponified in order to obtain a product of formula (I_{E}): 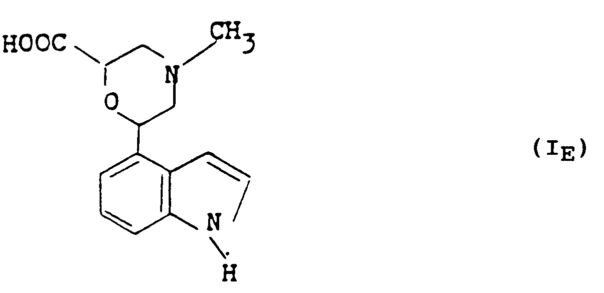 which either is isolated and, if desired, salified, or is reacted with an amine of formula (V): 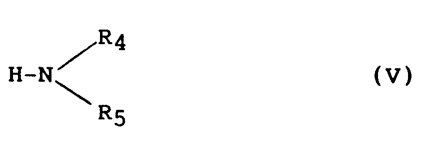 in which R₄ and R₅ represent a linear alkyl radical containing 1 to 4 carbon atoms, in order to obtain a product of formula (IF): 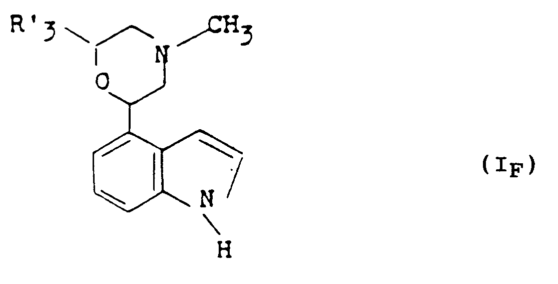 in which R'₃ represents a carboxy radical amidified by the amide of formula (V) defined above, which is isolated and if desired salified; or the said product of formula (I_{A}) is reduced in order to obtain a product of formula (I_{G}): 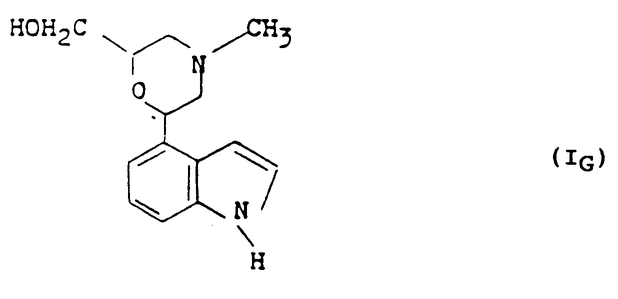 which either is isolated and, if desired, salified, or is reacted with methane chloride or paratoluene sulphonyl chloride, in order to obtain a product of formula (VI): 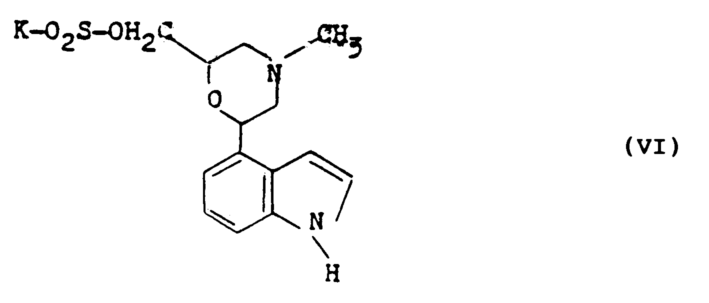 in which K represents a methyl or p-tolyl radical, which is reacted with an alkylmercaptan or with an alkaline cyanide, in order to obtain a product of formula (I_{H}): 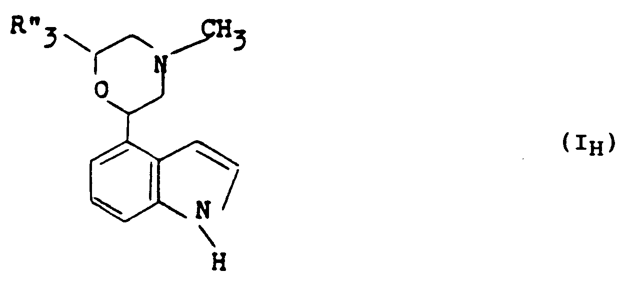 in which R"₃ represents an alkylthiomethyl or cyanomethyl radical, which is isolated and if desired salified, then if desired one or more of the reactions already indicated above for the products of formula (I_{A}) are carried out on the products of formulae (I_{E}), (I_{E}), (l_{G}) and (I_{H}), in order to obtain the corresponding products of formula (I), then the products thus obtained are isolated and, if desired, salified.

2. Process according to claim 1, characterized in that derivatives are prepared corresponding to formula (I) of claim 1, in which R represents a hydrogen atom, as well as their addition salts with mineral or organic acids.

3. Process according to claim 1 or 2, characterized in that derivatives are prepared corresponding to formula (I), in which R₂ represents a hydroxymethyl radical or an alkylthiomethyl radical the alkyl of which contains 1 to 3 carbon atoms or a cyanomethyl radical or a carboxy radical optionally amidified by an amine of formula 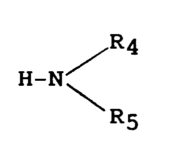 in which R₄ and R₅ represent a linear alkyl radical containing 1 to 4 carbon atoms, as well as their addition salts with mineral or organic acids.

4. Process according to claim 3, characterized in that derivatives are prepared corresponding to formula (I), in which R₂ represents an alkylthiomethyl radical the alkyl of which contains 1 to 3 carbon atoms, as well as their addition salts with mineral or organic acids.

5. Process according to any one of claims 1 to 4, characterized in that 4-/(2R-cis)-4-methyl-6-/(methylthio)-methyl/-2-morphinyl/-1 H-indole is prepared, as well as its addition salts with mineral or organic acids.

## Claims (Claims for the following Contracting State(s) : ES)

1. Preparation process for derivatives of 4-morpholinyl-1 H-indole, as well as their addition salts with mineral or organic acids, corresponding to general formula (I): 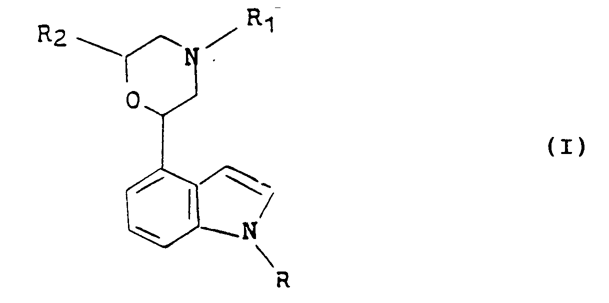 in which R represents a hydrogen atom or an alkyl radical containing 1 to 4 carbon atoms, R₁ represents a hydrogen atom, an alkyl radical containing 1 to 4 carbon atoms, R₂ represents a hydroxymethyl radical or an alkylthiomethyl radical the alkyl of which contains 1 to 3 carbon atoms, a cyanomethyl radical or a carboxy radical optionally esterified by an aliphatic alcohol containing 1 to 5 carbon atoms, or amidified by an amine of formula 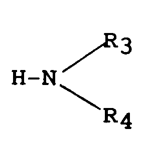 in which R₃ represents a hydrogen atom or an alkyl radical containing 1 to 4 carbon atoms, and R₄ represents an alkyl radical containing 1 to 4 carbon atoms, characterized in that a derivative of formula (II): 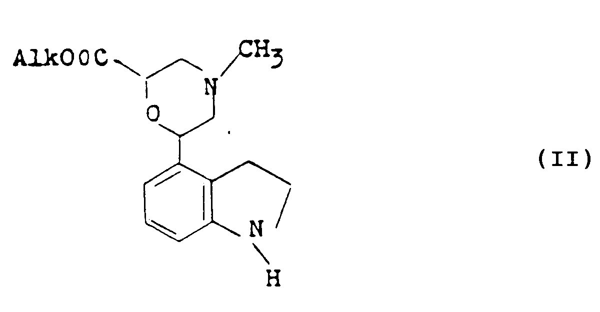 in which Alk represents an alkyl radical containing 1 to 4 carbon atoms, is subjected to a dehydrogenation in order to obtain a derivative of formula (I_{A}): 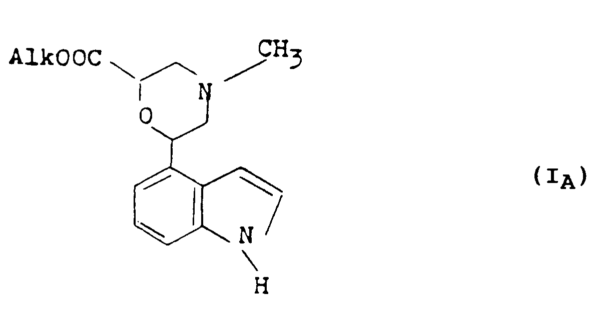 in which Alk has the meaning already indicated, which
- either is isolated and if desired, salified;
- or is demethylated in order to obtain the product of formula (I_{B}): 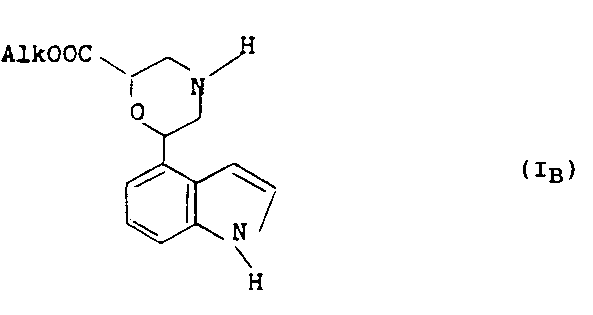 in which Alk has the meaning already indicated, which
- either is isolated and, if desired, salified;
- or is reacted with an alkylation agent, in order to obtain a product of formula (Iₑ): 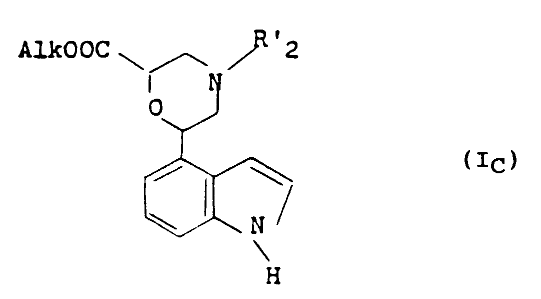 in which R'₂ has the meaning of R₂ already indicated with the exception of hydrogen and Alk has the meaning already indicated, which either is isolated and, if desired, salified, or is reacted with an alkaline amide, then with an alkyl halide of formula (IV): 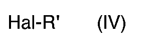 in which Hal represents a chlorine, bromine or iodine atom and R' represents an alkyl radical containing 1 to 4 carbon atoms, in order to obtain a product of formula (l_{D}): 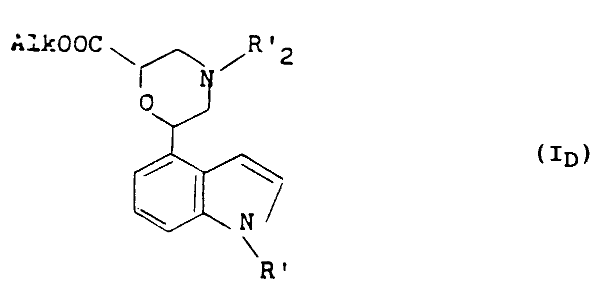 in which Alk, R' and R'₂ have the meaning already indicated, which is isolated and, if desired, salified;
- or the said product of formula (I_{A}) is saponified in order to obtain a product of formula (I_{E}): 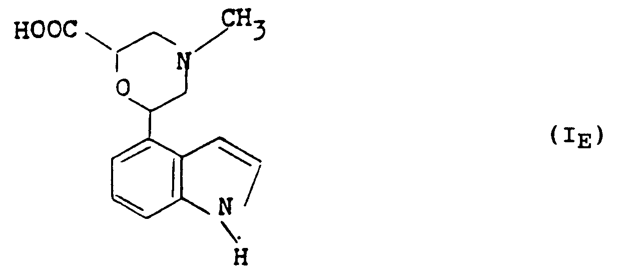 which either is isolated and, if desired, salified, or is reacted with an amine of formula (V): 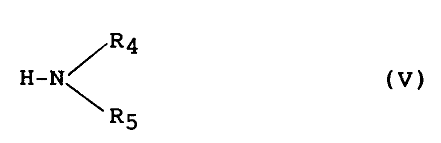 in which R₄ and R₅ represent a linear alkyl radical containing 1 to 4 carbon atoms, in order to obtain a product of formula (IF): 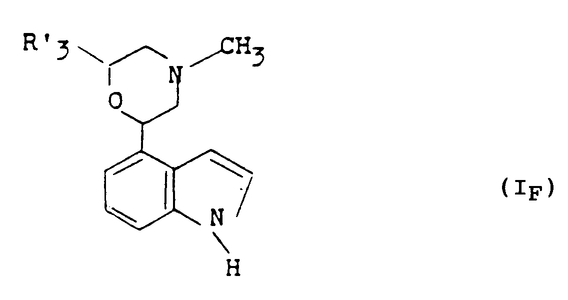 in which R'₃ represents a carboxy radical amidified by the amide of formula (V) defined above, which is isolated and if desired salified; or the said product of formula (I_{A}) is reduced in order to obtain a product of formula (I_{G}): 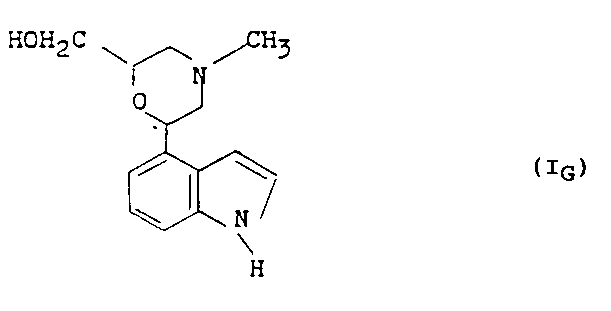 which either is isolated and, if desired, salified, or is reacted with methane chloride or paratoluene sulphonyl chloride, in order to obtain a product of formula (VI): 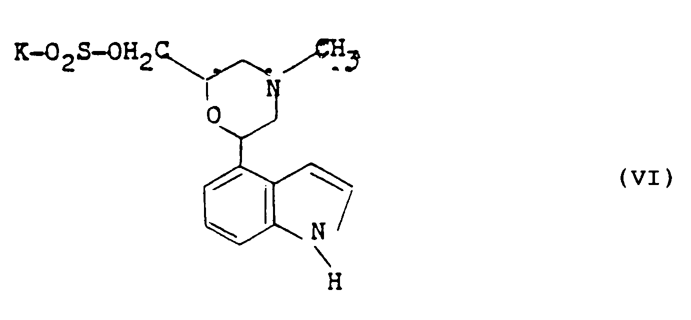 in which K represents a methyl or p-tolyl radical, which is reacted with an alkylmercaptan or with an alkaline cyanide, in order to obtain a product of formula (I_{H}): 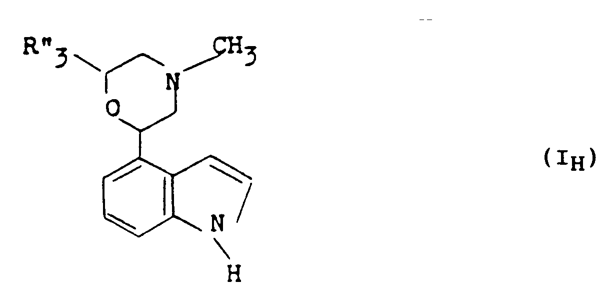 in which R"₃ represents an alkylthiomethyl or cyanomethyl radical, which is isolated and if desired salified, then if desired one or more of the reactions already indicated above for the products of formula (I_{A}) are carried out on the products of formulae (I_{E}), (IF), (l_{G}) and (I_{H}), in order to obtain the corresponding products of formula (I), then the products thus obtained are isolated and, if desired, salified.

2. Process according to claim 1, characterized in that derivatives are prepared corresponding to formula (I) of claim 1, in which R represents a hydrogen atom, as well as their addition salts with mineral or organic acids.

3. Process according to claim 1 or 2, characterized in that derivatives are prepared corresponding to formula (I), in which R₂ represents a hydroxymethyl radical or an alkylthiomethyl the alkyl of which contains 1 to 3 carbon atoms or a cyanomethyl radical or a carboxy radical optionally amidified by an amine of formula 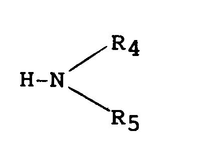 in which R₄ and R₅ represent a linear alkyl radical containing 1 to 4 carbon atoms, as well as their addition salts with mineral or organic acids.

4. Process according to claim 3, characterized in that derivatives are prepared corresponding to formula (I), in which R₂ represents an alkylthiomethyl radical the alkyl of which contains 1 to 3 carbon atoms, as well as their addition salts with mineral or organic acids.

5. Process according to any one of claims 1 to 4, characterized in that 4-/(2R-cis)-4-methyl-6-/(methylthio)-methyl/-2-morphinyl/-1 H-indole is prepared, as well as its addition salts with mineral or organic acids.

## Claims (Claims for the following Contracting State(s) : GR)

1. Preparation process for derivatives of 4-morpholinyl-1 H-indole, as well as their addition salts with mineral or organic acids, corresponding to general formula (I): 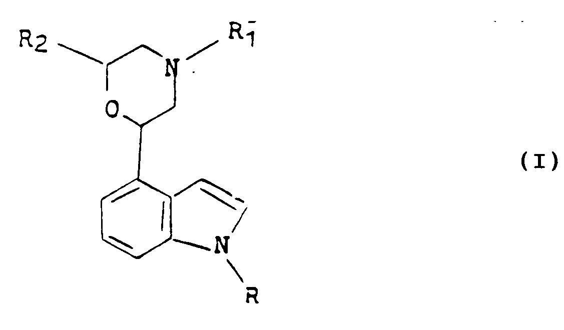 in which R represents a hydrogen atom or an alkyl radical containing 1 to 4 carbon atoms, R₁ represents a hydrogen atom, an alkyl radical containing 1 to 4 carbon atoms, R₂ represents a hydroxymethyl radical or an alkylthiomethyl radical the alkyl of which contains 1 to 3 carbon atoms, a cyanomethyl radical or a carboxy radical optionally esterified by an aliphatic alcohol containing 1 to 5 carbon atoms, or amidified by an amine of formula 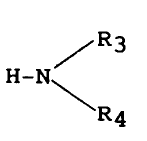 in which R₃ represents a hydrogen atom or an alkyl radical containing 1 to 4 carbon atoms, and R₄ represents an alkyl radical containing 1 to 4 carbon atoms, characterized in that a derivative of formula (II): 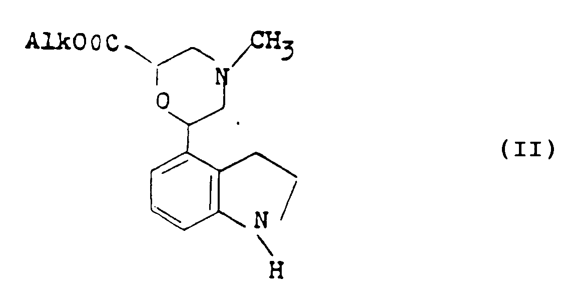 in which Alk represents an alkyl radical containing 1 to 4 carbon atoms, is subjected to a dehydrogenation in order to obtain a derivative of formula (I_{A}): 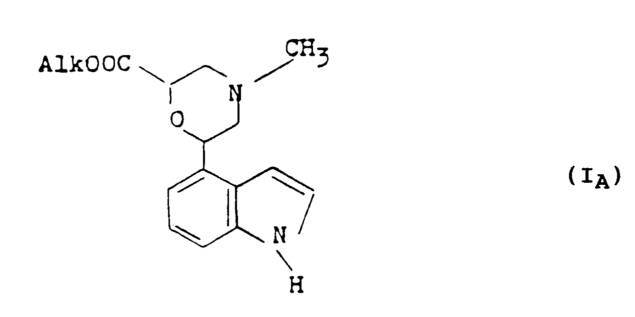 in which Alk has the meaning already indicated, which
- either is isolated and if desired, salified;
- or is demethylated in order to obtain the product of formula (I_{B}): 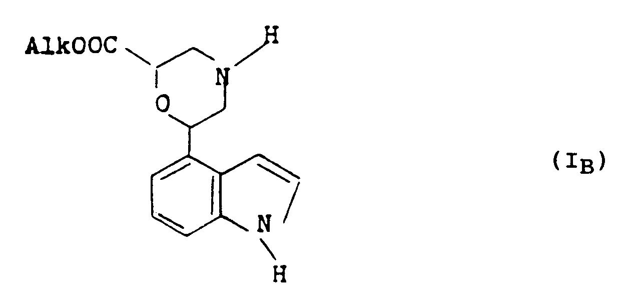 in which Alk has the meaning already indicated, which
- either is isolated and, if desired, salified;
- or is reacted with an alkylation agent, in order to obtain a product of formula (Iₑ): 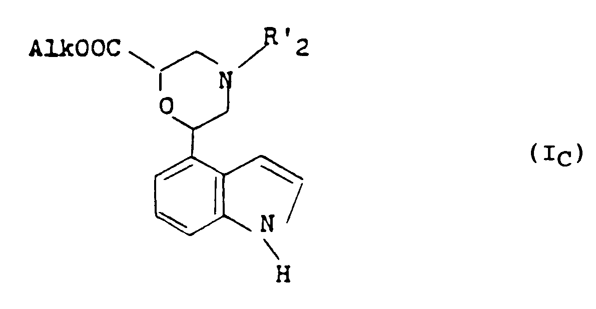 in which R'₂ has the meaning of R₂ already indicated with the exception of hydrogen and Alk has the meaning already indicated, which either is isolated and, if desired, salified, or is reacted with an alkaline amide, then with an alkyl halide of formula (IV): 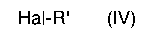 in which Hal represents a chlorine, bromine or iodine atom and R' represents an alkyl radical containing 1 to 4 carbon atoms, in order to obtain a product of formula (l_{D}): 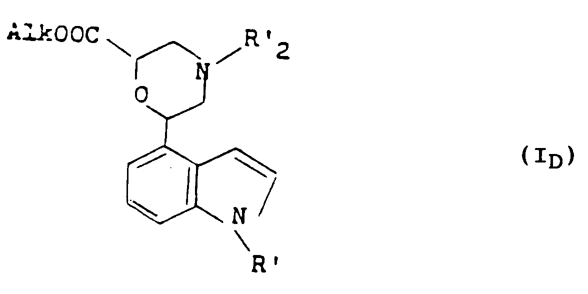 in which Alk, R' and R'₂ have the meaning already indicated, which is isolated and, if desired, salified;
- or the said product of formula (I_{A}) is saponified in order to obtain a product of formula (I_{E}): 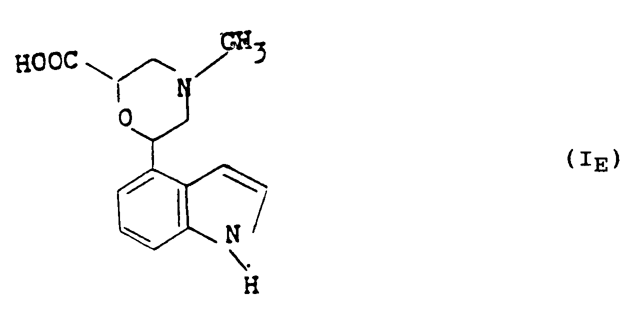 which either is isolated and, if desired, salified, or is reacted with an amine of formula (V): 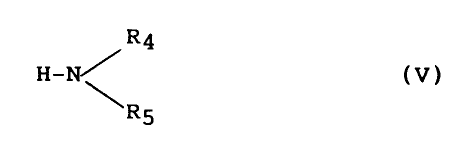 in which R₄ and R₅ represent a linear alkyl radical containing 1 to 4 carbon atoms, in order to obtain a product of formula (IF): 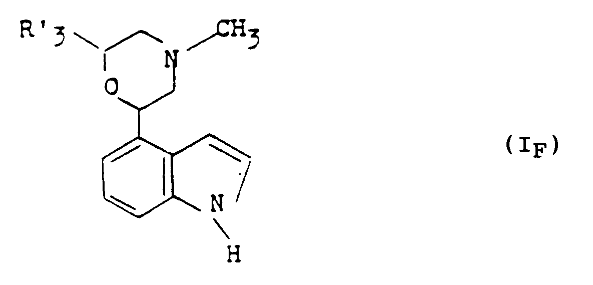 in which R'₃ represents a carboxy radical amidified by the amide of formula (V) defined above, which is isolated and if desired salified; or the said product of formula (I_{A}) is reduced in order to obtain a product of formula (I_{G}): 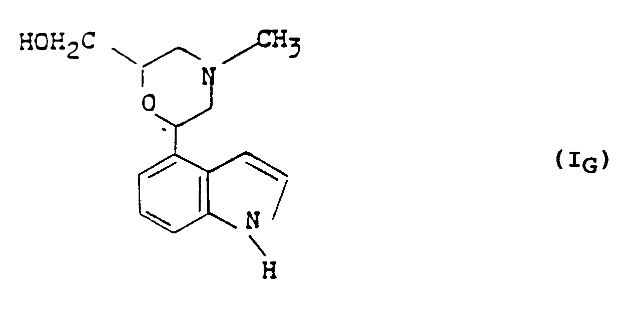 which either is isolated and, if desired, salified, or is reacted with methane chloride or paratoluene sulphonyl chloride, in order to obtain a product of formula (VI): 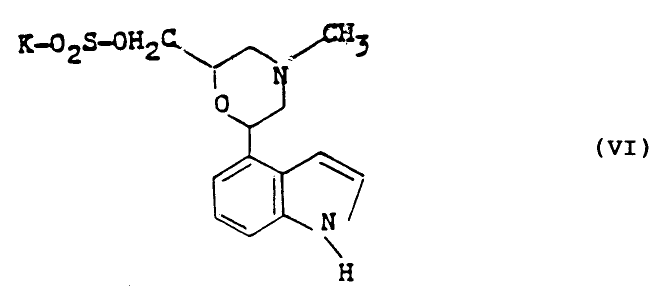 in which K represents a methyl or p-tolyl radical, which is reacted with an alkylmercaptan or with an alkaline cyanide, in order to obtain a product of formula (I_{H}): 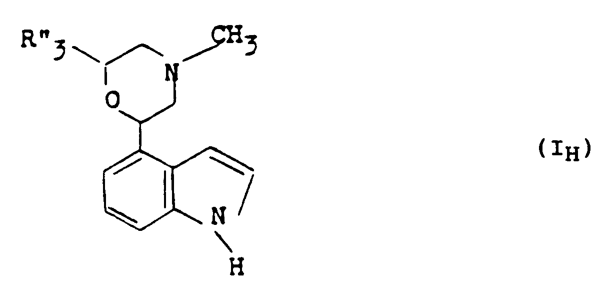 in which R"₃ represents an alkylthiomethyl or cyanomethyl radical, which is isolated and if desired salified, then if desired one or more of the reactions already indicated above for the products of formula (I_{A}) are carried out on the products of formulae (I_{E}), (IF), (l_{G}) and (I_{H}), in order to obtain the corresponding products of formula (I), then the products thus obtained are isolated and, if desired, salified.

2. Process according to claim 1, characterized in that derivatives are prepared corresponding to formula (I) of claim 1, in which R represents a hydrogen atom, as well as their addition salts with mineral or organic acids.

3. Process according to claim 1 or 2, characterized in that derivatives are prepared corresponding to formula (I), in which R₂ represents a hydroxymethyl radical or an alkylthiomethyl the alkyl of which contains 1 to 3 carbon atoms or a cyanomethyl radical or a carboxy radical optionally amidified by an amine of formula 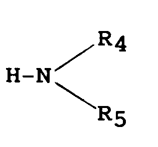 in which R₄ and R₅ represent a linear alkyl radical containing 1 to 4 carbon atoms, as well as their addition salts with mineral or organic acids.

4. Process according to claim 3, characterized in that derivatives are prepared corresponding to formula (I), in which R₂ represents an alkylthiomethyl radical the alkyl of which contains 1 to 3 carbon atoms, as well as their addition salts with mineral or organic acids.

5. Process according to any one of claims 1 to 4, characterized in that 4-/(2R-cis)-4-methyl-6-/(methylthio)-methyl/-2-morphinyl/-1 H-indole is prepared, as well as its addition salts with mineral or organic acids.

## Patentansprüche (Patentansprüche für folgende(n) Vertragsstaat(en) : BE, CH, DE, GB, IT, LI, LU, NL, SE)

1. 4-Morpholinyl-1 H-indol-Derivate sowie deren Additionssalze mit Mineral- oder organischen Säuren, dadurch gekennzeichnet, daß sie der allgemeinen Formel (I) 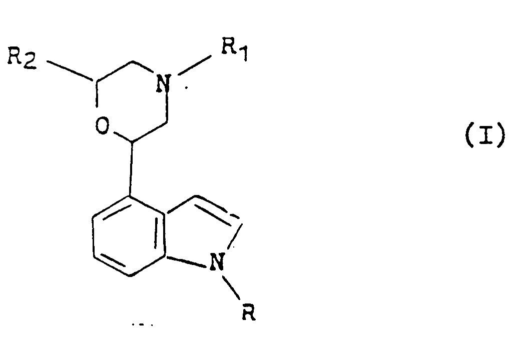 entsprechen, worin R für ein Wasserstoffatom oder einen Alkylrest mit 1 bis 4 Kohlenstoffatomen steht, R₁ ein Wasserstoffatom, einen Alkylrest mit 1 bis 4 Kohlenstoffatomen bedeutet, R₂ einen Hydroxymethylrest, einen Alkylthiomethylrest, dessen Alkylteil 1 bis 3 Kohlenstoffatome umfaßt, einen Cyanomethylrest oder eine Carboxygruppe, die gegebenenfalls durch einen aliphatischen Alkohol mit 1 bis 5 Kohlenstoffatomen verestert oder durch ein Amin der Formel 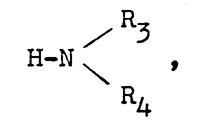 worin R₃ für ein Wasserstoffatom oder einen Alkylrest mit 1 bis 4 Kohlenstoffatomen steht, amidiert ist, wiedergibt und R₄ einen Alkylrest mit 1 bis 4 Kohlenstoffatomen bedeutet.

2. 4-Morpholinyl-1 H-indol-Derivate sowie deren Additionssalze mit Mineral- oder organischen Säuren, dadurch gekennzeichnet, daß sie der Formel (I) gemäß Anspruch 1 entsprechen, worin R für ein Wasserstoffatom steht.

3. 4-Morpholinyl-1 H-indol-Derivate sowie deren Additionssalze mit Mineral- oder organischen Säuren, dadurch gekennzeichnet, daß sie der Formel (I) gemäß Anspruch 2 entsprechen, worin R₂ einen Hydroxymethylrest, einen Alkylthiomethylrest, dessen Alkylteil 1 bis 3 Kohlenstoffatome umfaßt, oder einen Cyanomethylrest oder einen Carboxyrest bedeutet, der gegebenenfalls durch ein Amin der Formel 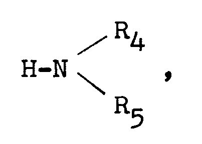 worin R₄ und R₅ für einen linearen Alkylrest mit 1 bis 4 Kohlenstoffatomen stehen, amidiert ist.

4. 4-Morpholinyl-1 H-indol-Derivate gemäß Anspruch 3 sowie deren Additionssalze mit Mineral- oder organischen Säuren, dadurch gekennzeichnet, daß R₂ einen Alkylthiomethylrest bedeutet, dessen Alkylteil 1 bis 3 Kohlenstoffatome umfaßt.

5. 4-[(2R-cis)-4-Methyl-6-[(methylthio)-methyl]-2-morpholinyl]-1 H-indol sowie dessen Additionssalze mit Mineral- oder organischen Säuren.

6. Verfahren zur Herstellung der 4-Morpholinyl-1 H-indol-Derivate der Formel I gemäß Anspruch 1 sowie von deren Additionssalzen mit Mineral- oder organischen Säuren, dadurch gekennzeichnet, daß man ein Derivat der Formel (ll) 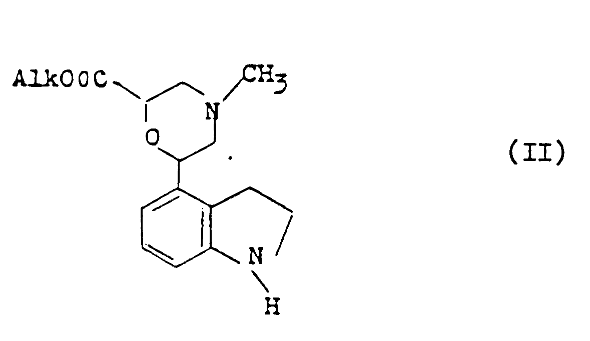 worin Alk für einen Alkylrest mit 1 bis 4 Kohlenstoffatomen steht, einer Dehydrierung unterzieht, um zu einem Derivat der Formel (I_{A}) 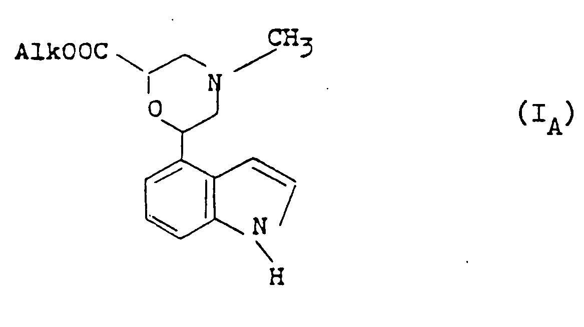 worin Alk die vorstehend angegebene Bedeutung besitzt, zu gelangen, welches man
- entweder isoliert und gewünschtenfalls in ein Salz überführt;
- oder demethyliert, um zu dem Produkt der Formel (l_{B}) 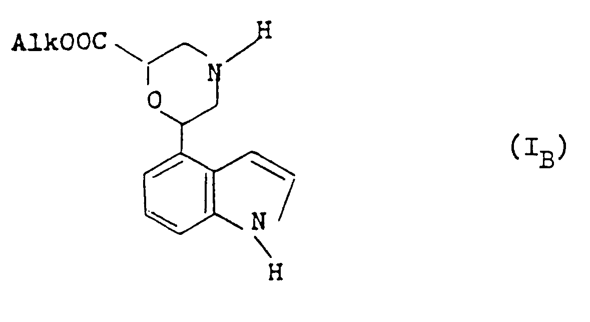 worin Alk die vorstehend angegebene Bedeutung besitzt, zu gelangen, welches man
- entweder isoliert und gewünschtenfalls in ein Salz überführt;
- oder mit einem Alkylierungsmittel umsetzt, um zu einem Produkt der Formel (l_{c}) 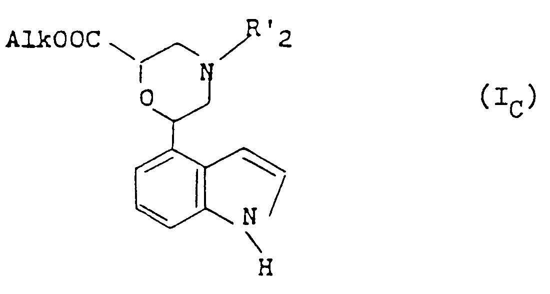 worin R'₂ die in Anspruch 1 für R₂ angegebene Bedeutung mit Ausnahme von Wasserstoff besitzt und Alk die vorstehend angegebene Bedeutung aufweist, zu gelangen, welches man entweder isoliert und gewünschtenfalls in ein Salz überführt oder mit einem Alkaliamid, danach mit einem Alkylhalogenid der Formel (IV) 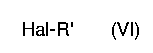 worin Hal für ein Chlor-, Brom- oder Jodatom steht und R' einen Alkylrest mit 1 bis 4 Kohlenstoffatomen bedeutet, umsetzt, um zu einem Produkt der Formel (l_{D}) 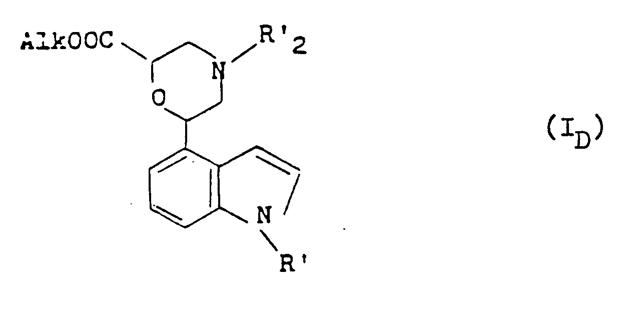 worin Alk, R' und R'₂ die vorstehend angegebene Bedeutung besitzen, zu gelangen, welches man isoliert und gewünschtenfalls in ein Salz überführt;
- oder man das Produkt der Formel (I_{A}) verseift, um ein Produkt der Formel (l_{E}) 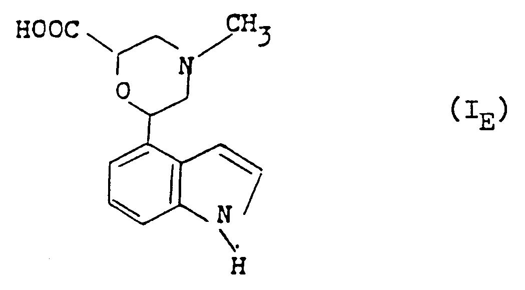 zu erhalten, welches man entweder isoliert und gewünschtenfalls in ein Salz überführt, oder mit einem Amin der Formel (V) 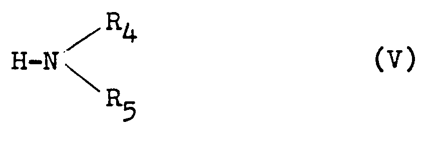 worin R₄ und R₅ die in Anspruch 3 angegebene Bedeutung besitzen, umsetzt, um zu einem Produkt der Formel (I_{F}) 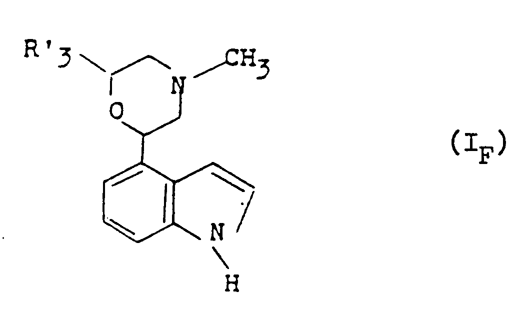 worin R'₃ einen durch das Amid der Formel (V), wie vorstehend definiert, amidierten Carboxyrest bedeutet, zu gelangen, welches man isoliert und gewünschtenfalls in ein Salz überführt;
- oder das Produkt der Formel (I_{A}) reduziert, um zu einem Produkt der Formel (l_{G}) 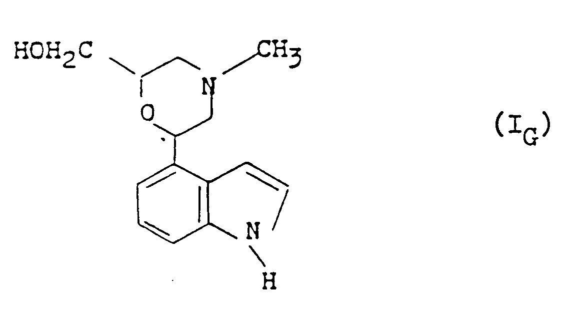 zu gelangen, welches man entweder isoliert und gewünschtenfalls in ein Salz überführt oder mit Methan- oder p-Toluolsulfonylchlorid umsetzt, um zu einem Produkt der Formel (VI) 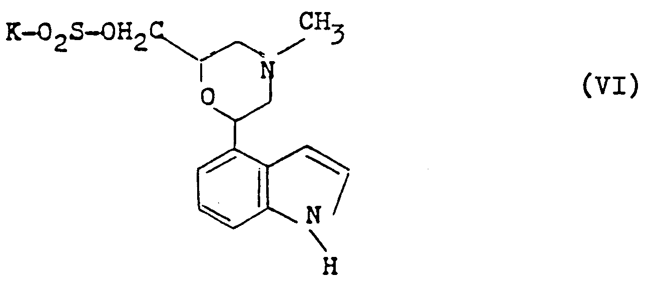 worin K für einen Methyl- oder p-Tolylrest steht, zu gelangen, welches man mit einem Alkylmercaptan oder mit einem Alkalicyanid umsetzt, um zu einem Produkt der Formel (l_{H}) 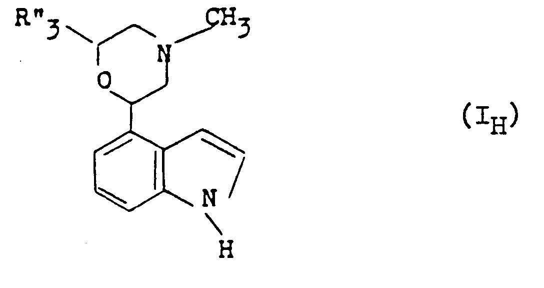 worin R"₃ einen Alkylthiomethylrest oder einen Cyanomethylrest bedeutet, zu gelangen, welches man isoliert und gewünschtenfalls in ein Salz überführt, hiernach dann gewünschtenfalls an den Produkten der Formeln (l_{E}), (I_{F}), (l_{G}) und (l_{H}) eine oder mehrere der vorstehend für die Produkte der Formel (I_{A}) angegebenen Reaktionen vornimmt, um zu den entsprechenden, unter die Formel (I) fallenden Produkten zu gelangen, und dann die so erhaltenen Produkte isoliert und gewünschtenfalls in ein Salz überführt.

7. Arzneimittel, dadurch gekennzeichnet, daß sie aus den neuen 4-Morpholinyl-1H-indol-Derivatender Formel (I) gemäß Anspruch 1 sowie aus deren Additionssalzen mit pharmazeutisch verträglichen Säuren bestehen.

8. Arzneimittel, dadurch gekennzeichnet, daß sie aus den neuen 4-Morpholinyl-1 H-indol-Derivaten gemäß Anspruch 2 sowie aus deren Additionssalzen mit pharmazeutisch verträglichen Säuren bestehen.

9. Arzneimittel, dadurch gekennzeichnet, daß sie aus den neuen 4-Morpholinyl-1 H-indol-Derivaten gemäß den Ansprüchen 3 oder 4 sowie aus deren Additionssalzen mit pharmazeutisch verträglichen Säuren bestehen.

10. Arznemittel, dadurch gekennzeichnet, daß sie aus den 4-Morpholinyl-1H-indol-Derivatengemäß Anspruch 5 sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel gemäß einem der Ansprüche 7 bis 10 enthalten.

12. Derivate der Formel (XIII) 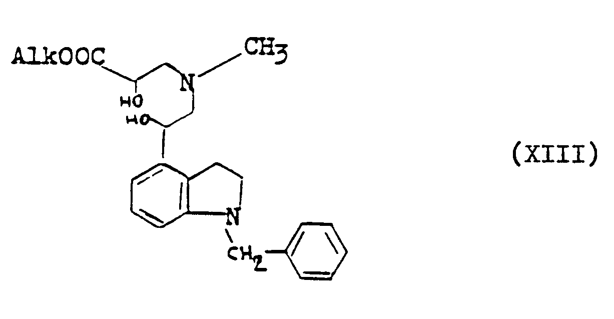 worin Alk für einen Alkylrest mit 1 bis 4 Kohlenstoffatomen steht.

13. Derivate der Formel (XIV) 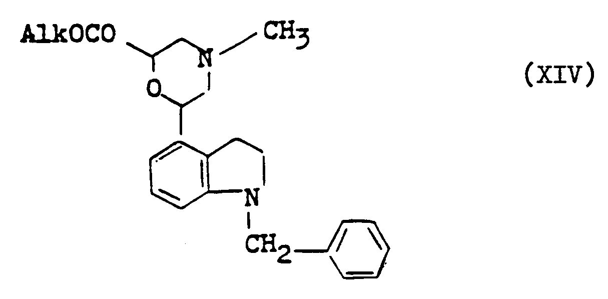 worin Alk für einen Alkylrest mit 1 bis 4 Kohlenstoffatomen steht.

## Patentansprüche (Patentansprüche für folgende(n) Vertragsstaat(en) : AT)

1. Verfahren zur Herstellung von 4-Morpholinyl-1 H-indolderivaten sowie von deren Additionssalzen mit Mineral- oder organischen Säuren der allgemeinen Formel (I) 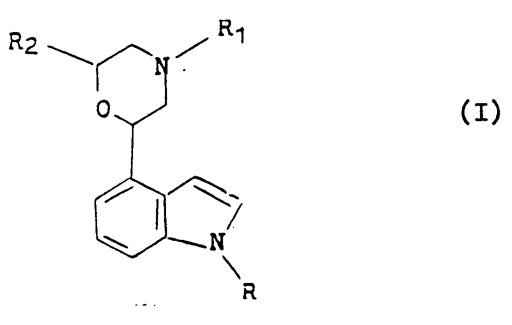 worin R für ein Wasserstoffatom oder einen Alkylrest mit 1 bis 4 Kohlenstoffatomen steht, R₁ ein Wasserstoffatom, einen Alkylrest mit 1 bis 4 Kohlenstoffatomen bedeutet, R₂ einen Hydroxymethylrest, einen Alkylthiomethylrest, dessen Alkylteil 1 bis 3 Kohlenstoffatome umfaßt, einen Cyanomethylrest oder eine Carboxygruppe, die gegebenenfalls durch einen aliphatischen Alkohol mit 1 bis 5 Kohlenstoffatomen verestert oder durch ein Amin der Formel 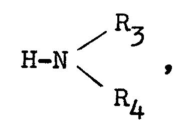 worin R₃ für ein Wasserstoffatom oder einen Alkylrest mit 1 bis 4 Kohlenstoffatomen steht, amidiert ist, wiedergibt und R₄ einen Alkylrest mit 1 bis 4 Kohlenstoffatomen bedeutet, dadurch gekennzeichnet, daß man ein Derivat der Formel (ll) 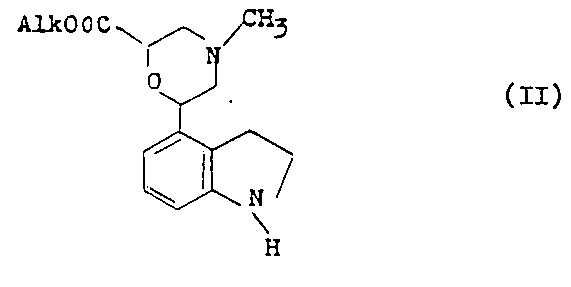 worin Alk für einen Alkylrest mit 1 bis 4 Kohlenstoffatomen steht, einer Dehydrierung unterzieht, um zu einem Derivat der Formel (I_{A}) 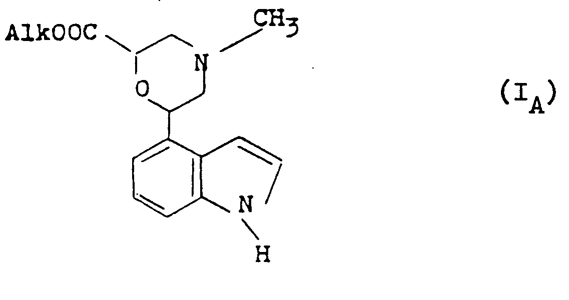 worin Alk die vorstehend angegebene Bedeutung besitzt, zu gelangen, welches man
- entweder isoliert und gewünschtenfalls in ein Salz überführt;
- oder demethyliert, um zu dem Produkt der Formel (l_{B}) 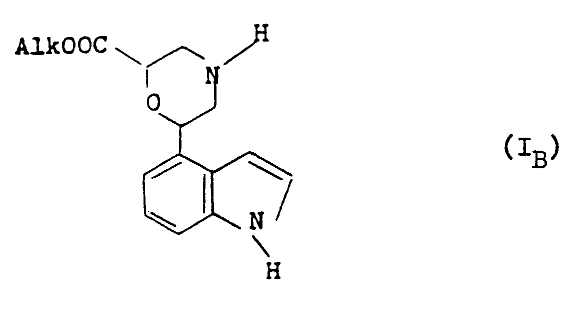 worin Alk die vorstehend angegebene Bedeutung besitzt, zu gelangen, welches man
- entweder isoliert und gewünschtenfalls in ein Salz überführt;
- oder mit einem Alkylierungsmittel umsetzt, um zu einem Produkt der Formel (l_{c}) 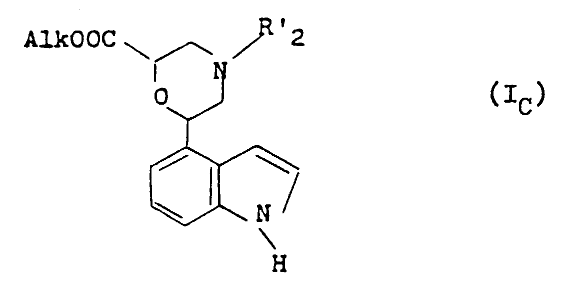 worin R'₂ die vorstehend für R₂ angegebene Bedeutung mit Ausnahme von Wasserstoff besitzt und Alk die vorstehend angegebene Bedeutung aufweist, zu gelangen, welches man entweder isoliert und gewünschtenfalls in ein Salz überführt oder mit einem Alkaliamid, danach mit einem Alkylhalogenid der Formel (IV) 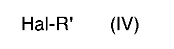 worin Hal für ein Chlor-, Brom- oder Jodatom steht und R' einen Alkylrest mit 1 bis 4 Kohlenstoffatomen bedeutet, umsetzt, um zu einem Produkt der Formel (l_{D}) 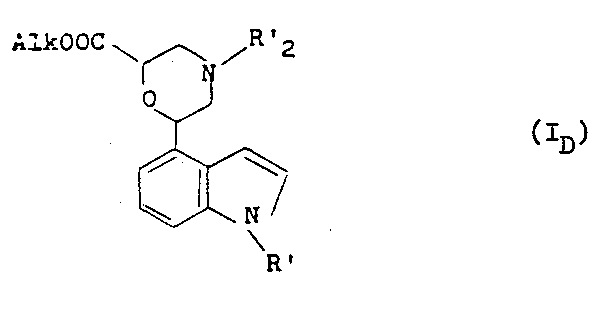 worin Alk, R' und R'₂ die vorstehend angegebene Bedeutung besitzen, zu gelangen, welches man isoliert und gewünschtenfalls in ein Salz überführt;
- oder man das Produkt der Formel (I_{A}) verseift, um ein Produkt der Formel (l_{E}) 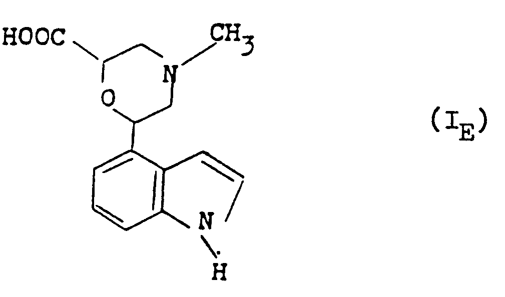 zu erhalten, welches man entweder isoliert und gewünschtenfalls in ein Salz überführt, oder mit einem Amin der Formel (V) 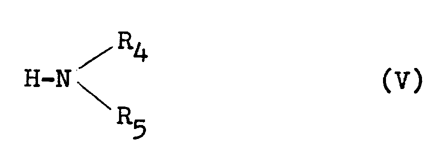 worin R₄ und R₅ für einen linearen Alkylrest mit 1 bis 4 Kohlenstoffatomen stehen, umsetzt, um zu einem Produkt der Formel (I_{F}) 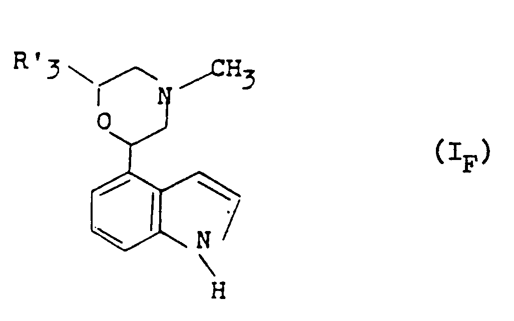 worin R'₃ einen durch das Amid der Formel (V), wie vorstehend definiert, amidierten Carboxyrest bedeutet, zu gelangen, welches man isoliert und gewünschtenfalls in ein Salz überführt;
- oder das Produkt der Formel (I_{A}) reduziert, um zu einem Produkt der Formel (l_{G}) 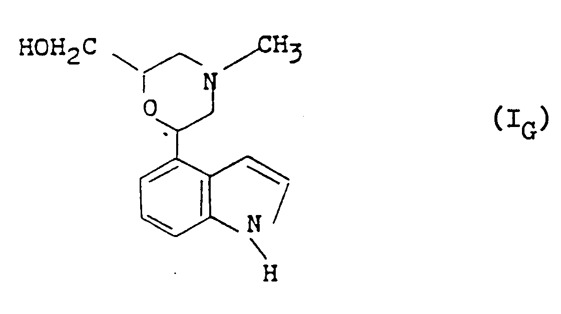 zu gelangen, welches man entweder isoliert und gewünschtenfalls in ein Salz überführt oder mit Methan- oder p-Toluolsulfonylchlorid umsetzt, um zu einem Produkt der Formel (VI) 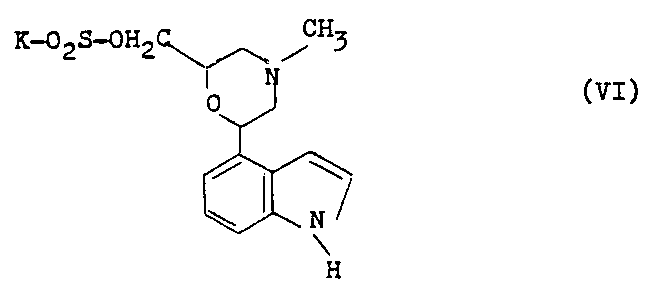 worin K für einen Methyl- oder p-Tolylrest steht, zu gelangen, welches man mit einem Alkylmercaptan oder mit einem Alkalicyanid umsetzt, um zu einem Produkt der Formel (l_{H}) 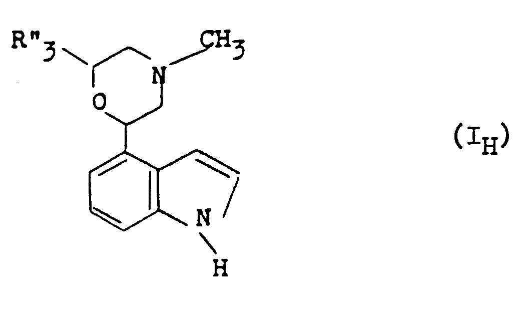 worin R"₃ einen Alkylthiomethylrest oder einen Cyanomethylrest bedeutet, zu gelangen, welches man isoliert und gewünschtenfalls in ein Salz überführt, hiernach dann gewünschtenfalls an den Produkten der Formeln (l_{E}), (I_{F}), (l_{G}) und (l_{H}) eine oder mehrere der vorstehend für die Produkte der Formel (I_{A}) angegebenen Reaktionen vornimmt, um zu den entsprechenden, unter die Formel (I) fallenden Produkten zu gelangen, und dann die so erhaltenen Produkte isoliert und gewünschtenfalls in ein Salz überführt.

2. Verfahren gemäß Anspruch 1, dadurch gekennzeichnet, daß man die der Formel (I) von Anspruch 1 entsprechenden Derivate, worin R für ein Wasserstoffatom steht, sowie deren Additionssalze mit Mineral- oder organischen Säuren herstellt.

3. Verfahren gemäß Anspruch 1 oder 2, dadurch gekennzeichnet, daß man die der Formel (I) entsprechenden Derivate, worin R₂ einen Hydroxymethylrest, einen Alkylthiomethylrest, dessen Alkylteil 1 bis 3 Kohlenstoffatome umfaßt, oder einen Cyanomethylrest oder einen Carboxyrest bedeutet, der gegebenenfalls durch ein Amin der Formel 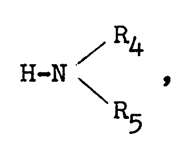 worin R₄ und R₅ für einen linearen Alkylrest mit 1 bis 4 Kohlenstoffatomen stehen, amidiert ist, sowie deren Additionssalze mit Mineral- oder organischen Säuren herstellt.

4. Verfahren gemäß Anspruch 3, dadurch gekennzeichnet, daß man die der Formel (I) entsprechenden Derivate, worin R₂ einen Alkylthiomethylrest wiedergibt, dessen Alkylteil 1 bis 3 Kohlenstoffatome umfaßt, sowie deren Additionssalze mit Mineral- oder organischen Säuren herstellt.

5. Verfahren gemäß einem der Ansprüche 1 bis 4, dadurch gekennzeichnet, daß man das 4-[(2R-cis)-4-Methyl-6-[(methylthio)-methyl]-2-morpholinyl]-1 H-indol sowie dessen Additionssalze mit Mineral- oder organischen Säuren herstellt.

## Patentansprüche (Patentansprüche für folgende(n) Vertragsstaat(en) : ES)

1. Verfahren zur Herstellung von 4-Morpholinyl-1H-indolderivatensowie von deren Additionssalzen mit Mineral- oder organischen Säuren der allgemeinen Formel (I) 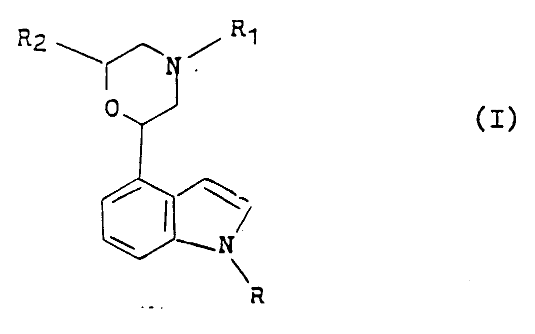 worin R für ein Wasserstoffatom oder einen Alkylrest mit 1 bis 4 Kohlenstoffatomen steht, R₁ ein Wasserstoffatom, einen Alkylrest mit 1 bis 4 Kohlenstoffatomen bedeutet, R₂ einen Hydroxymethylrest, einen Alkylthiomethylrest, dessen Alkylteil 1 bis 3 Kohlenstoffatome umfaßt, einen Cyanomethylrest oder eine Carboxygruppe, die gegebenenfalls durch einen aliphatischen Alkohol mit 1 bis 5 Kohlenstoffatomen verestert oder durch ein Amin der Formel 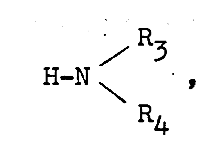 worin R₃ für ein Wasserstoffatom oder einen Alkylrest mit 1 bis 4 Kohlenstoffatomen steht, amidiert ist, wiedergibt und R₄ einen Alkylrest mit 1 bis 4 Kohlenstoffatomen bedeutet, dadurch gekennzeichnet, daß man ein Derivat der Formel (11) 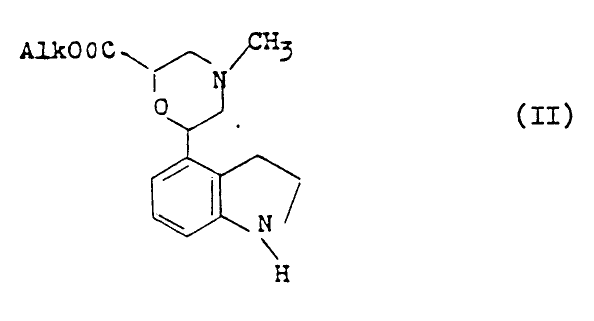 worin Alk für einen Alkylrest mit 1 bis 4 Kohlenstoffatomen steht, einer Dehydrierung unterzieht, um zu einem Derivat der Formel (I_{A}) 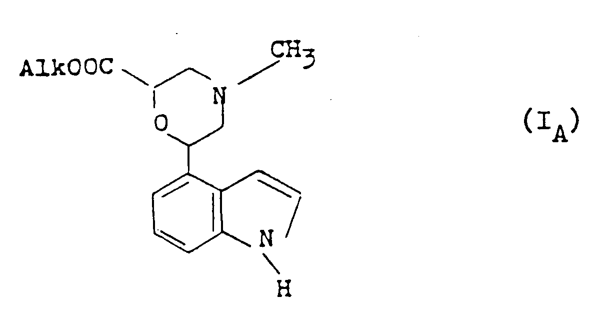 worin Alk die vorstehend angegebene Bedeutung besitzt, zu gelangen, welches man
- entweder isoliert und gewünschtenfalls in ein Salz überführt;
- oder demethyliert, um zu dem Produkt der Formel (l_{B}) 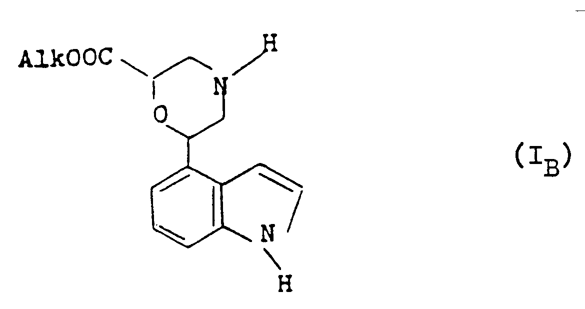 worin Alk die vorstehend angegebene Bedeutung besitzt, zu gelangen, welches man
- entweder isoliert und gewünschtenfalls in ein Salz überführt;
- oder mit einem Alkylierungsmittel umsetzt, um zu einem Produkt der Formel (l_{c}) 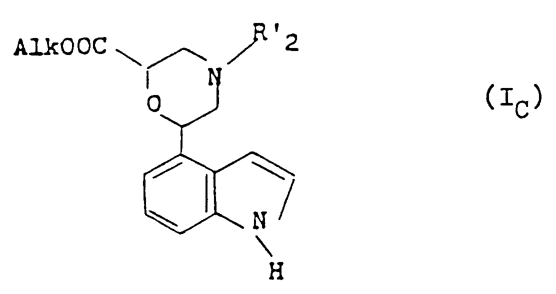 worin R'₂ die vorstehend für R₂ angegebene Bedeutung mit Ausnahme von Wasserstoff besitzt und Alk die vorstehend angegebene Bedeutung aufweist, zu gelangen, welches man entweder isoliert und gewünschtenfalls in ein Salz überführt oder mit einem Alkaliamid, danach mit einem Alkylhalogenid der Formel (IV) 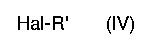 worin Hal für ein Chlor-, Brom- oder Jodatom steht und R' einen Alkylrest mit 1 bis 4 Kohlenstoffatomen bedeutet, umsetzt, um zu einem Produkt der Formel (l_{D}) 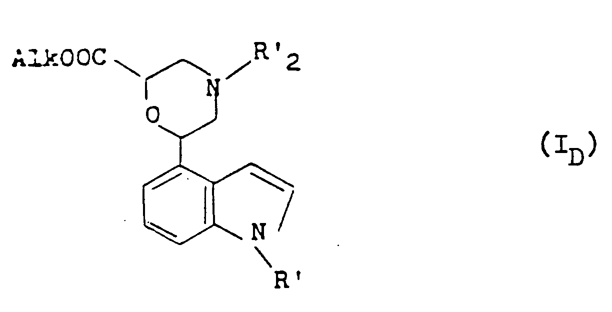 worin Alk, R' und R'₂ die vorstehend angegebene Bedeutung besitzen, zu gelangen, welches man isoliert und gewünschtenfalls in ein Salz überführt;
- oder man das Produkt der Formel (I_{A}) verseift, um ein Produkt der Formel (l_{E}) 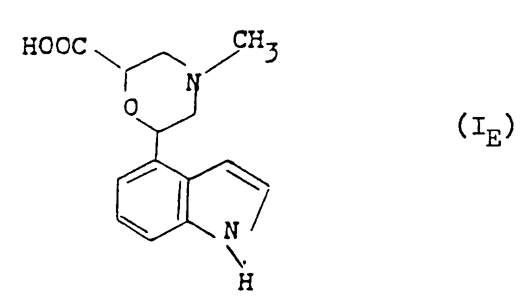 zu erhalten, welches man entweder isoliert und gewünschtenfalls in ein Salz überführt, oder mit einem Amin der Formel (V) 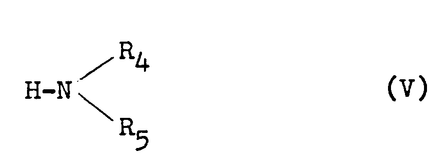 worin R₄ und R₅ für einen linearen Alkylrest mit 1 bis 4 Kohlenstoffatomen stehen, umsetzt, um zu einem Produkt der Formel (I_{F}) 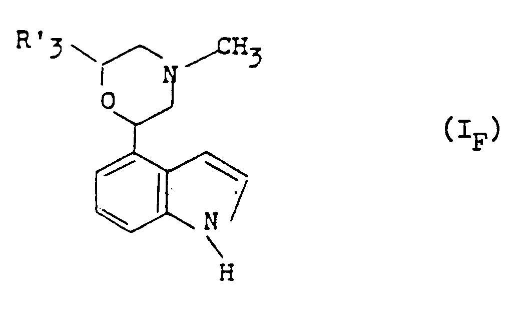 worin R'₃ einen durch das Amid der Formel (V), wie vorstehend definiert, amidierten Carboxyrest bedeutet, zu gelangen, welches man isoliert und gewünschtenfalls in ein Salz überführt;
- oder das Produkt der Formel (I_{A}) reduziert, um zu einem Produkt der Formel (l_{G}) 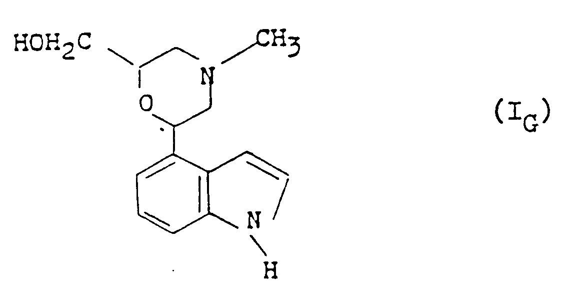 zu gelangen, welches man entweder isoliert und gewünschtenfalls in ein Salz überführt oder mit Methan- oder p-Toluolsulfonylchlorid umsetzt, um zu einem Produkt der Formel (VI) 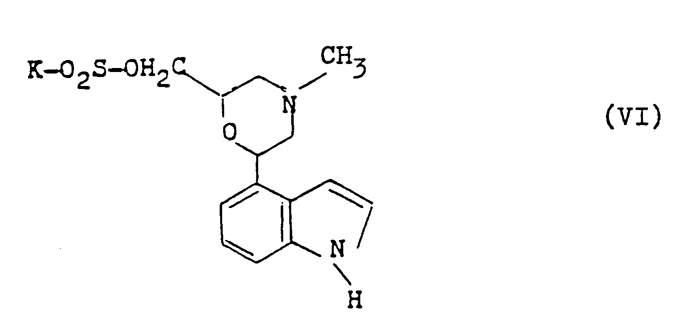 worin K für einen Methyl- oder p-Tolylrest steht, zu gelangen, welches man mit einem Alkylmercaptan oder mit einem Alkalicyanid umsetzt, um zu einem Produkt der Formel (l_{H}) 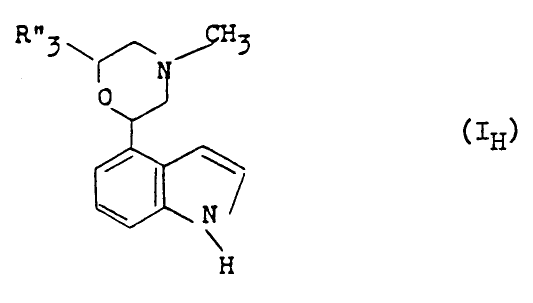 worin R"₃ einen Alkylthiomethylrest oder einen Cyanomethylrest bedeutet, zu gelangen, welches man isoliert und gewünschtenfalls in ein Salz überführt, hiernach dann gewünschtenfalls an den Produkten der Formeln (l_{E}), (I_{F}), (l_{G}) und (l_{H}) eine oder mehrere der vorstehend für die Produkte der Formel (I_{A}) angegebenen Reaktionen vornimmt, um zu den entsprechenden, unter die Formel (I) fallenden Produkten zu gelangen, und dann die so erhaltenen Produkte isoliert und gewünschtenfalls in ein Salz überführt.

2. Verfahren gemäß Anspruch 1, dadurch gekennzeichnet, daß man die der Formel (I) von Anspruch 1 entsprechenden Derivate, worin R für ein Wasserstoffatom steht, sowie deren Additionssalze mit Mineral- oder organischen Säuren herstellt.

3. Verfahren gemäß Anspruch 1 oder 2, dadurch gekennzeichnet, daß man die der Formel (I) entsprechenden Derivate, worin R₂ einen Hydroxymethylrest, einen Alkylthiomethylrest, dessen Alkylteil 1 bis 3 Kohlenstoffatome umfaßt, oder einen Cyanomethylrest oder einen Carboxyrest bedeutet, der gegebenenfalls durch ein Amin der Formel 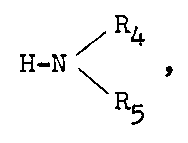 worin R₄ und R₅ für einen linearen Alkylrest mit 1 bis 4 Kohlenstoffatomen stehen, amidiert ist, sowie deren Additionssalze mit Mineral- oder organischen Säuren herstellt.

4. Verfahren gemäß Anspruch 3, dadurch gekennzeichnet, daß man die der Formel (I) entsprechenden Derivate, worin R₂ einen Alkylthiomethylrest wiedergibt, dessen Alkylteil 1 bis 3 Kohlenstoffatome umfaßt, sowie deren Additionssalze mit Mineral- oder organischen Säuren herstellt.

5. Verfahren gemäß einem der Ansprüche 1 bis 4, dadurch gekennzeichnet, daß man das 4-[(2R-cis)-4-Methyl-6-[(methylthio)-methyl]-2-morpholinyl]-1 H-indol sowie dessen Additionssalze mit Mineral- oder organischen Säuren herstellt.

## Patentansprüche (Patentansprüche für folgende(n) Vertragsstaat(en) : GR)

1. Verfahren zur Herstellung von 4-Morpholinyl-1 H-indolderivaten sowie von deren Additionssalzen mit Mineral- oder organischen Säuren der allgemeinen Formel (I) 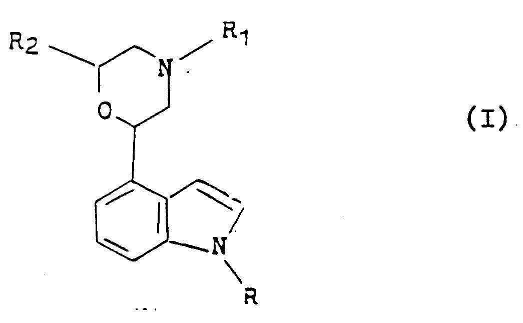 worin R für ein Wasserstoffatom oder einen Alkylrest mit 1 bis 4 Kohlenstoffatomen steht, R₁ ein Wasserstoffatom, einen Alkylrest mit 1 bis 4 Kohlenstoffatomen bedeutet, R₂ einen Hydroxymethylrest, einen Alkylthiomethylrest, dessen Alkylteil 1 bis 3 Kohlenstoffatome umfaßt, einen Cyanomethylrest oder eine Carboxygruppe, die gegebenenfalls durch einen aliphatischen Alkohol mit 1 bis 5 Kohlenstoffatomen verestert oder durch ein Amin der Formel 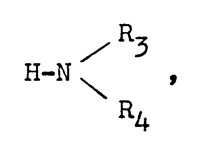 worin R₃ für ein Wasserstoffatom oder einen Alkylrest mit 1 bis 4 Kohlenstoffatomen steht, amidiert ist, wiedergibt und R₄ einen Alkylrest mit 1 bis 4 Kohlenstoffatomen bedeutet, dadurch gekennzeichnet, daß man ein Derivat der Formel (II) 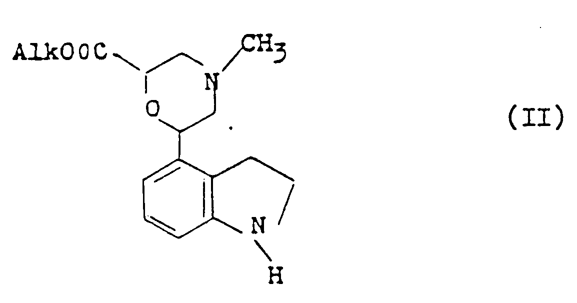 worin Alk für einen Alkylrest mit 1 bis 4 Kohlenstoffatomen steht, einer Dehydrierung unterzieht, um zu einem Derivat der Formel (I_{A}) 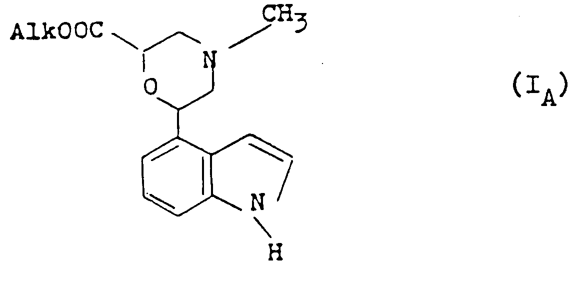 worin Alk die vorstehend angegebene Bedeutung besitzt, zu gelangen, welches man
- entweder isoliert und gewünschtenfalls in ein Salz überführt;
- oder demethyliert, um zu dem Produkt der Formel (l_{B}) 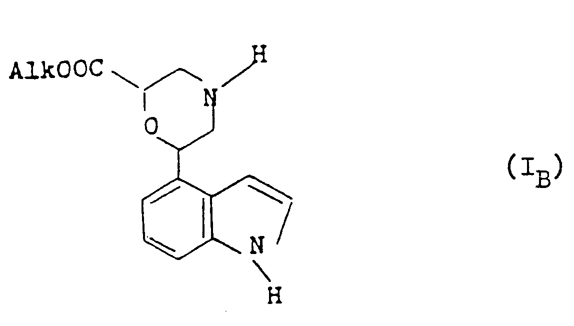 worin Alk die vorstehend angegebene Bedeutung besitzt, zu gelangen, welches man
- entweder isoliert und gewünschtenfalls in ein Salz überführt;
- oder mit einem Alkylierungsmittel umsetzt, um zu einem Produkt der Formel (l_{c}) 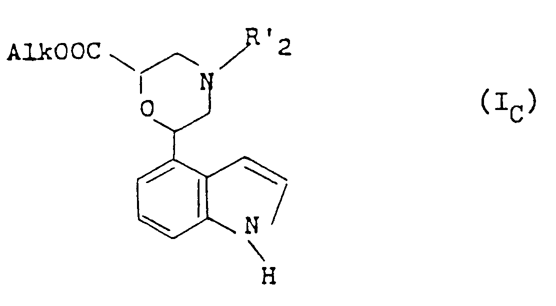 worin R'₂ die vorstehend für R₂ angegebene Bedeutung mit Ausnahme von Wasserstoff besitzt und Alk die vorstehend angegebene Bedeutung aufweist, zu gelangen, welches man entweder isoliert und gewünschtenfalls in ein Salz überführt oder mit einem Alkaliamid, danach mit einem Alkylhalogenid der Formel (IV) 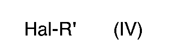 worin Hal für ein Chlor-, Brom- oder Jodatom steht und R' einen Alkylrest mit 1 bis 4 Kohlenstoffatomen bedeutet, umsetzt, um zu einem Produkt der Formel (l_{D}) 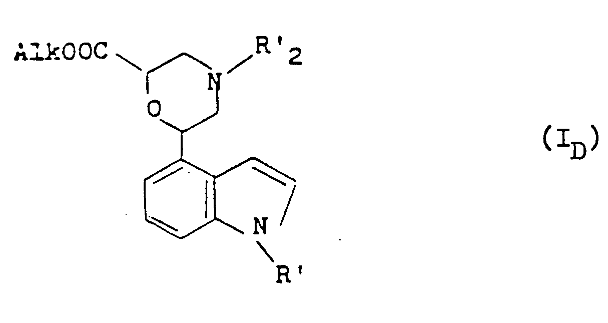 worin Alk, R' und R'₂ die vorstehend angegebene Bedeutung besitzen, zu gelangen, welches man isoliert und gewünschtenfalls in ein Salz überführt;
- oder man das Produkt der Formel (I_{A}) verseift, um ein Produkt der Formel (l_{E}) 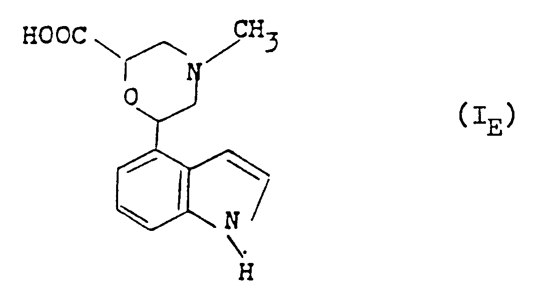 zu erhalten, welches man entweder isoliert und gewünschtenfalls in ein Salz überführt, oder mit einem Amin der Formel (V) 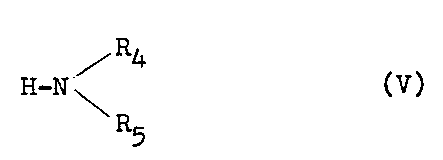 worin R₄ und R₅ für einen linearen Alkylrest mit 1 bis 4 Kohlenstoffatomen stehen, umsetzt, um zu einem Produkt der Formel (I_{F}) 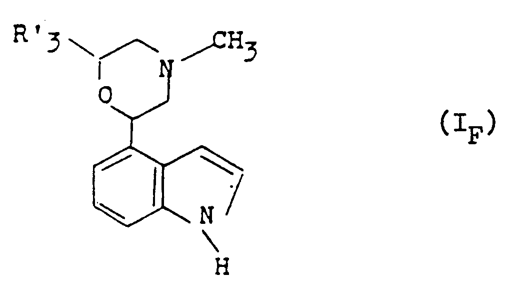 worin R'₃ einen durch das Amid der Formel (V), wie vorstehend definiert, amidierten Carboxyrest bedeutet, zu gelangen, welches man isoliert und gewünschtenfalls in ein Salz überführt;
- oder das Produkt der Formel (I_{A}) reduziert, um zu einem Produkt der Formel (l_{G}) 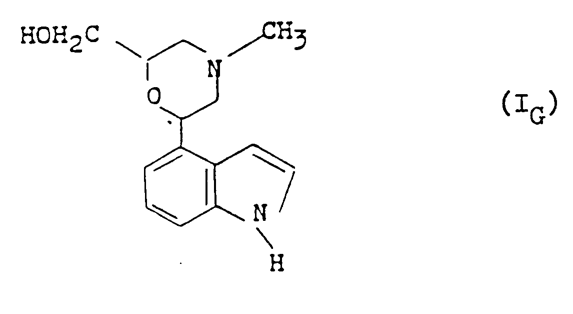 zu gelangen, welches man entweder isoliert und gewünschtenfalls in ein Salz überführt oder mit Methan- oder p-Toluolsulfonylchlorid umsetzt, um zu einem Produkt der Formel (VI) 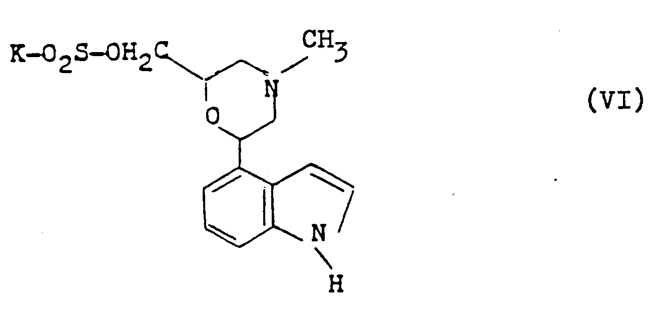 worin K für einen Methyl- oder p-Tolylrest steht, zu gelangen, welches man mit einem Alkylmercaptan oder mit einem Alkalicyanid umsetzt, um zu einem Produkt der Formel (l_{H}) 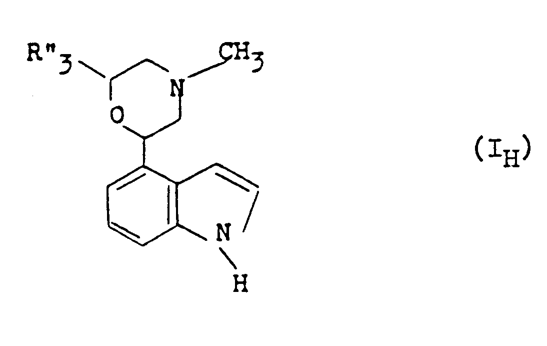 worin R"₃ einen Alkylthiomethylrest oder einen Cyanomethylrest bedeutet, zu gelangen, welches man isoliert und gewünschtenfalls in ein Salz überführt, hiernach dann gewünschtenfalls an den Produkten der Formeln (l_{E}), (I_{F}), (l_{G}) und (l_{H}) eine oder mehrere der vorstehend für die Produkte der Formel (I_{A}) angegebenen Reaktionen vornimmt, um zu den entsprechenden, unter die Formel (I) fallenden Produkten zu gelangen, und dann die so erhaltenen Produkte isoliert und gewünschtenfalls in ein Salz überführt.

2. Verfahren gemäß Anspruch 1, dadurch gekennzeichnet, daß man die der Formel (I) von Anspruch 1 entsprechenden Derivate, worin R für ein Wasserstoffatom steht, sowie deren Additionssalze mit Mineral- oder organischen Säuren herstellt.

3. Verfahren gemäß Anspruch 1 oder 2, dadurch gekennzeichnet, daß man die der Formel (I) entsprechenden Derivate, worin R₂ einen Hydroxymethylrest, einen Alkylthiomethylrest, dessen Alkylteil 1 bis 3 Kohlenstoffatome umfaßt, oder einen Cyanomethylrest oder einen Carboxyrest bedeutet, der gegebenenfalls durch ein Amin der Formel 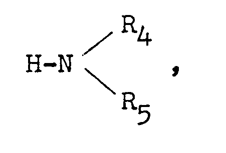 worin R₄ und R₅ für einen linearen Alkylrest mit 1 bis 4 Kohlenstoffatomen stehen, amidiert ist, sowie deren Additionssalze mit Mineral- oder organischen Säuren herstellt.

4. Verfahren gemäß Anspruch 3, dadurch gekennzeichnet, daß man die der Formel (I) entsprechenden Derivate, worin R₂ einen Alkylthiomethylrest wiedergibt, dessen Alkylteil 1 bis 3 Kohlenstoffatome umfaßt, sowie deren Additionssalze mit Mineral- oder organischen Säuren herstellt.

5. Verfahren gemäß einem der Ansprüche 1 bis 4, dadurch gekennzeichnet, daß man das 4-[(2R-cis)-4-Methyl-6-[(methylthio)-methyl]-2-morpholinyl]-1 H-indol sowie dessen Additionssalze mit Mineral- oder organischen Säuren herstellt.
